# EUROPEAN PATENT SPECIFICATION

(11) **EP 2 129 799 B1**
(45) Date of publication and mention of the grant of the patent: **20.06.2012**
(21) Application number: 08733628.5
(22) Date of filing: 19.03.2008
(51) Int. Cl.: C12Q 1/68, C12Q 1/02, G01N 33/53, G01N 33/68, C07K 16/18, C07K 14/47, C12N 15/12

(54) **METHODS OF STRATIFYING ADOLESCENT IDIOPATHIC SCOLIOSIS, ISOLATED NUCLEIC ACID MOLECULES FOR USE IN SAME**
VERFAHREN ZUR STRATIFIZIERUNG VON ADOLESZENTER IDIOPATHISCHER SKOLIOSE, ISOLIERTE NUKLEINSÄUREMOLEKÜLE ZUR VERWENDUNG DAFÜR
PROCÉDÉS DE STRATIFICATION D'UNE SCOLIOSE IDIOPATHIQUE DE L'ADOLESCENCE, MOLÉCULES D'ACIDE NUCLÉIQUE ISOLÉES EN VUE D'UNE UTILISATION DANS LESDITS PROCÉDÉS

(30) Priority: 19.03.2007 US 895490 P; 28.03.2007 US 908417 P
(43) Date of publication of application: 09.12.2009
(73) Proprietor: Chu Sainte-justine, Montreal QC H3T 1C5 (CA)
(72) Inventor: MOREAU, Alain, Montréal, Québec H1A 5T5 (CA)
(74) Representative: Grosset-Fournier, Chantal Catherine
(86) International application number: PCT/CA2008/000524
(87) International publication number: WO 2008/113174

(56) References cited:
- WO-A1-03/073102
- MARCIL A ET AL: "Pitx1 and Pitx2 are required for development of hindlimb buds" DEVELOPMENT, COMPANY OF BIOLOGISTS, CAMBRIDGE, GB, vol. 130, no. 1, 1 January 2003 (2003-01-01), pages 45-55, XP008109650 ISSN: 0950-1991
- GIAMPIETRO P F ET AL: "An analysis of PAX1 in the development of vertebral malformations" CLINICAL GENETICS, vol. 68, no. 5, November 2005 (2005-11), pages 448-453, XP002567757 ISSN: 0009-9163
- PICARD C. ET AL.: 'New emerging role of Pitx1 transcription factor in osteoarthritis pathogenesis' CLINICAL ORTHOPAEDIC AND RELATED RESEARCH vol. 462, September 2007, pages 59 - 66, XP008110290
- OSAMURA R. ET AL.: 'Expression of Ptx1 in the adult rat pituitary glands and pituitary cell lines-Hormones secreting cells and folliculo-stellate (FS) cells' JOURNAL OF HISTOCHEMISTRY AND CYTOCHEMISTRY vol. 47, December 1999, page 1648C-1648 + ABSTR. NO. 26, XP008119036
- MOREAU A. ET AL.: 'Melatonin signaling dysfunction in adolescent idiopathic scoliosis' SPINE vol. 29, no. 16, 15 August 2004, pages 1772 - 1781, XP009129278
- JOHNSTON J. ET AL.: 'Gonadotrophin-releasing hormone drives melatonin receptor down-regulation in the developing pituitary gland' PROCEEDINGS OF NATIONAL ACADEMY OF SCIENCE vol. 100, no. 5, 04 March 2003, pages 2831 - 2835, XP008119037
- MILLER N. ET AL.: 'Linkage analysis of genetic loci for kyphoscoliosis on chromosomes 5p13, 13q13.3, and 13q32' AMERICAN JOURNAL OF MEDICAL GENETICS PART A vol. 140, 15 May 2006, pages 1059 - 1068, XP008119039
- BELL M. ET AL.: 'Pelvic skeleton reduction and Pitx1 expression in threespine stickleback populations' NOVARTIS FOUNDATION SYMPOSIUM vol. 284, 2007, pages 225 - 244, XP009129281

### SEQUENCE LISTING

<110> CHU Sainte-Justine
   Moreau, Alain
<120> METHODS OF STRATIFYING ADOLESCENT IDIOPATHIC SCOLIOSIS, ISOLATED NUCLEIC ACID MOLECULES FOR USE IN SAME AND KITS USING
<130> 14033.35
<140> US 60/908,417
   <141> 2007-03-28
<150> US 60/895,490
   <151> 2007-03-19
<160> 27
<170> PatentIn version 3.3
<210> 1
   <211> 10006
   <212> DNA
   <213> HomoSapiens
<400> 1
   cccaaattgt ctatctgtga tagtggctgt gccccttcgg gccctgagca ccctgtgtcc      60
   tgtgcagcag tcagatatct ggagggagac tgaggcactg gctgcagagc ttgtgatcat     120
   gagagagact cactaggact acagatgggt aaactgaggc cttcgagggg gcagctccag     180
   aaaggcaggg gccataatgt ctcaccttca tatttcccgt gccaagctgt ggccttctgc     240
   attcatggca gatgagtgga caaaggctga tggactgatg gagaaacaaa gggatagatg     300
   gagcagctgg gcagctcagc aaatgatgct gcaatgatct gcttccaact cacctcaaat     360
   ccatccttct ctctccaggc agagtgggct tttaagatac acatctggcc aggtctctca     420
   ctgttcaaac ccttcatctg ctcctttttg ccttcaggat aacatcccac cctcctatca     480
   aggactatgg agccctgtgg gatctggttc ccacttgatt ctccaacttc ctcttcccct     540
   atgccctgcc ttctcatctg ttccagtgct attatgaagc cacacgttct tcctttatta     600
   tcaagcatac cacagtttat ctcacctcag aggctttgca cagtatattt tcctagggag     660
   gggtccccag gtggtagaaa aacggttaca gccaactcct ccatgtgtca ctcaagaccc     720
   ttcaacagca ggctgcagat ttcctctcca gcactgtgct ccaactgtgt aatggatttc     780
   tgtgtctgcc tccttgactc aacccaaatg aacaagagcc atctatctct gtatctctgc     840
   aatcacaggc acaaaatagg tgctctctac attttttcca acctgagagg ccattctaga     900
   agggtctcag gccacggttc tgtccagtat tccatgcaga tgctgacagg actgcaatta     960
   aaaaaatact tgagatgccc aaatgcccaa atagcttctc attttgcttt gactaccaat 1020
   aattgcacag tgcaatagaa taatgctcaa atacattaac atcttacttg atcctagggg 1080
   gtcctctcta cttttaaagc ctcaaacttc ctccctctca caggtgaaaa ggggagtaca 1140
   aatacattcc ctcccttgct ctgcggatcc attcctacag gtagtcaaga ctctgagctt 1200
   cccctctgac tttctggcag tgcttcacct ctccccacag atgagtgcag gaacaattct 1260
   aacagacttc agactcttcc aacagagcca atccctcccc atcacgttgg agtggacttg 1320
   ctacccacac catcaacagg cccctgagaa acttcacagg gcaggggctt ctttggtaaa 1380
   accaaccctt tccttccacc agctcagaga agttgttcca gatagatgcc aggattcttg 1440
   gaggagcatg gtgattctgg ggtggagcct ctgaccctgg ccaaccaaag gcctgagcca 1500
   ctcttccccc acaaagtgat tggcgcagga gtgggcatgt aagctggacc cagccaatca 1560
   gcataaccaa atcccttgcc caccctgggg agggtcctat agttgttgcc aagagaggct 1620
   cacccctgtt ctcaagaagc ttacaactgg taggggcaac aggttttaat ctatcattac 1680
   acaaatattt aatttcaata ctgttgcaat aagagctatg aaggaaaaga acactccccc 1740
   tacttttact tggtgttaag atttgaagaa gaaaaaaaaa aaacactgcc tgaaggattg 1800
   ttatggcctt ctatataata gtggctgcag acatttgccc attatgttca gcatgaaccc 1860
   atgtgacaaa ttcatcaaag cgttttgcac tagggagaaa aatttgtatt agaggaagca 1920
   cagcagtttg gactgaaaga caaagaaaat tcagccaatt ctgctgatct tttttgatgg 1980
   ggcacctgga agctgaaagc taaagtggta ctcaggaaca gggactgcta cttctgttcc 2040
   tggtgaatcc tgccccaaac ctcctctctc tctgattcct gattccactg tgccagtggg 2100
   aatatatgct ccccaagatg tcaaaactaa agggaaattg caaaaaatat atacatatat 2160
   ttttagagag aaaataagat tataaaaaat gtgttttgta ccccccaagt ttcactaaga 2220
   acttcctgac ttccaggccc tggttgtgcc ccacgcacca gcctgcccag ctttcctgga 2280
   ccaaacttcc tagcacctaa gcaggggatg agggcagata aactaaatca gaaaagggat 2340
   ctgttcctcc tagactcaac caacatgacc accgtgggga aagaagaaac aaaaacaaga 2400
   gcaaactctc ttaaagagca gcctggcagc tatcaccatt agcattcagc ccctctgtcc 2460
   acaggactca ggaccaaacc cctcaccttc actatcccat ccgtttccca agaagcagaa 2520
   atacttattc tcacatttca cagatgggga agctgaggct aggagaggtt atgttatttg 2580
   ccaaagccac acaactagta aaagccactg acaagattct ggctcaggcc atcaggtgcc 2640
   agaggcagca ttttttggca ccacaggccc tgcctgggaa caagagcatg cagaaaatct 2700
   cacaagagat gggaacaaaa tttggaaaat tgctagcgtg cagggagggg ggaaggtgtg 2760
   atttcctgct acagacgcca gagtaaaagc caccccagga gtgcctgtgc agccctccat 2820
   agtaaggtcc agcggctgca tttatgccca aagatgcccc tggtgcttgg agtggaagga 2880
   agattccaga gacaagatta gaaacttctc agcttagcag ctctagggct ggacccgcca 2940
   acaagccatt ttacacataa agcagtcaat gggagggggt agacgtaggg ggctaaactc 3000
   cccacagcac agggtccaag ttggtagact gcactttctc caggcgcagg tccgctagtg 3060
   ccggcatcgg ggactcgtta tcttaacttg cgaccctggg tgcacagagc cctgcacaca 3120
   ccactggaga ggggttcctg ctgtcgaggg ttgagaggag ggtatggagt ccctggaaca 3180
   gcacgacagg gtgcagaggc cacctggcag ggcctgaaca ccgaggcctc tgtgagcttg 3240
   ggtcgggccg gcttcccgct tcggaggttg gggagggggt cgtgggtctc tgcgttccca 3300
   ggccaagcgg ccctggaggc acggactagc aggacgccga ggtggcgcgg gtcgcggcct 3360
   ctcccgcagc agctgtcggc gagaaccagg cagggaggcg ccgctgctga ctgcatctca 3420
   tgaaagattc aggcccggct gccgcgctgc catctcccgg caccttgcgc cggaaacggt 3480
   cgctctggag cccgtggccg tcggcgggca ggcttagccg ctccagtccc tgagaaaggc 3540
   aggccacagc ccgacctgcc ctgtggtccc atcccataat cccaacagca agcaggctca 3600
   ggctgggcac ttcggggtac caggagtagg ttcggccaac tggtttccac catgaggctt 3660
   cgcgcacagg gttatctggc cacgaggcaa cgctggggag ccctgtggcc tgagggtggg 3720
   caaaggacag gctcccagtt cccttgcgtc cagcccgtct cccagcggca gccagccagg 3780
   aacggcctgc ggggccacag gggtggaggc gtcaccgttc gcaggcccgc agcaggatgg 3840
   tcgctggggg atgtgcaggc ataggggttg gacaaggggc cccagaagtg tctgtcctga 3900
   ggggttggtg tgccctttcc tcaccagccc agcccctgag gagagggaag aaggcaattc 3960
   ccccaaccag ggcaggtcgg gcggttgccc caccctaaca ccccctaccc ccaaacacag 4020
   aaaacctggg gtctgtcctc aaacctccct ggctgccacg ctctgggcag cggactctcc 4080
   ctgccaacac gcaagaccag ctccctcccg caggctgagc agaaagaaga aaggtccaat 4140
   ctcaaaaccc caaactcgac caccagcccc cgtctaaacg gaagtagggc cagcccctca 4200
   cgagtcaggt gggaaactgg gcccagggag agaaagtgcc ccagggccag gctgggaccc 4260
   gctctggctt cgtccgcatg cggcagggcc cctccacgga ggtcccaggg cgcgctcccc 4320
   ggcctcgagg cccggccgcc agccgcgcgg accccagcct acgccccgag ggaggccagg 4380
   acccctagcc ggcgggactg cgcgccgccc ctctccccgc aggtcccggc gaacacctag 4440
   cttcccctcc ccccaccctt cccgcctccc ggccagtgtc cccgccttcc ccgcgggcga 4500
   cgggcggcgg cggcgggagg agcgggccga gccgaggaag ccccggcctc gcgcgctggg 4560
   atgtagcgaa ccagcagggg ccgaagaacc gtgcagtgcc agagccagag ctggatccgg 4620
   ggccccagcc ggagccgaaa cctgagccag agtccgcggc gggcgagccc ggagcccacg 4680
   agccgcagac gcagcgctgc ccaggtgggg taagagacgc tgggctaggg gcgcagggtc 4740
   tccgcggtgg aggggcgcag ggaggtggcg gccgagtcct gcgcagtttg ctcctggcgt 4800
   gtgtgggtcc acccggcggc gcgggacagc gcaaggcgcg gaaggtcagg agccttcgag 4860
   gcagcgcgag gagctcgttc ctgcgcccag ggcacagtca tagccgccgt caccgggtgc 4920
   tacctcaccc aaccggcggg atcaaccctc tgctttggct ccgggcacct caagagggta 4980
   gcagcctcgg gggcacgggc cacggccccg cgaagggcac aacctgagaa gcccgtggca 5040
   gcccctcgca gcgtcgggtg acacagggct cccccacccc caggagaagt gggcaggaga 5100
   gagggccgcc cgctgctccc cgctgcgtcc agggatggag ggccccacca cccatggaat 5160
   tgctggcccc tctgcgtggc ccgggacttc agccgtggct tcgcgtcaag agggtatttt 5220
   tcctaaacga aaccgcttcg ttcgttcgtt cgttcgttcg ttcgggcagc aatgccgcag 5280
   aaaagcagca gacgtcggtc cgcgccctgg ctctcttcgc cccggacccc gacgtcccgc 5340
   cgcagcgctc ggaggtgccc ccagcccaag gcagcctgct ctcgccggca caggtcgggc 5400
   tttttcttcc caggagagaa accccaattc ccttcgtaac gtccaataaa gacattcccg 5460
   cggcttctcc caggtttggt tgttgacgca gggtcccgga gcacgcagtc gcttctcaag 5520
   aaccgggtct cggatttctg aaattgacca gcttcgtaaa ttggagccta ttctcccgcg 5580
   gcaaaggcag ggccccaaag ccgggatcgc agtaatggga accccaggct ggaatccggg 5640
   tcccaagctt ttccgattta ggaattcccc gaatctacaa atatttagtc cacttttctg 5700
   aaaaactaaa ttctgaaaaa cacaaattct cttgacatcc ctgtgacctc tgaaagccac 5760
   cagggccaga gggaggaaat cccaggttgc tgtccactgg gggaggattc aggtctaggg 5820
   ttcaggtcta cggtagtcag ggcaaaagct acaggcagca ggggcagcac aggagacttg 5880
   ctgtccccgt gccctttccc ggggctgctt tcggcctccc gcatctcttc cagggaaagg 5940
   aaaagaggtg ggctggggct tggagaccag gctgtctgga ctctaggatg cagaggcctc 6000
   cagacaggct cagggtgctc ttctcccatg aaagcagccg ctgggaggag gaggctatgg 6060
   tgcatccata agttgcccct ctgctcccca gttgtgcgac cagctgctac ctccttccta 6120
   gtcttcttcc ccacagctca gccattctca ggaaccagac agcgtccatg gacttaggtg 6180
   agagatgggc cgggtagagc catgggtcct accagccgct gactgagcgg cccacggcac 6240
   agagtcctga gttccatact cccatctgtg cctcactggc ggcagtcctg ctcaaataca 6300
   tcctggctct ccccgggaca ggctggggat ccccatttgg caggaagcct cagactgggg 6360
   tcccaggaag cctaaaggag ccagtgaggt ctttccagcc cctacctgag caccctcctc 6420
   cccacttacc cagtaattgc tgtattcaaa gaaacgggag cttttattgg ggagggggtg 6480
   ttagatcagg cagaaagagg taggtggtcc aaacctgcac tcccaaaaca gggttttcaa 6540
   gtttgaactt ctccacggac taagaggctt agggctggaa tgtcccagag agtcatggat 6600
   agccctggtg gcaggccatg gcacattcct tcctttttcc taaaatacct tgattctggg 6660
   agcaaggatt agggcacggt gcccccgtgg gtgggtagaa ggatgccccc ccactgagag 6720
   ccttccaacc acccttccca aattacatta ctaaaccatt cttgggcaca gggtgttttt 6780
   agtgagccag gcttcaggaa gggtcctcat ggtgactact tcaaccccac aacagcccaa 6840
   gctcttctgc tcagcccagc caagacccta aactccaaaa ttcttgaaaa tcagagaatc 6900
   attgctggct ttgtgtggtc acggaggggt ggggaacagg gcacatggtt ccagctccac 6960
   taagccccct tccctcctct cttcgtgtcc catcagcaag tgagctggat gctgaagcag 7020
   caggcagagt ccggtgttgg acatgggaac tgaggcacag tgcagatcaa gccttaacct 7080
   tgagggaaac acaggtcaca tagcacagct gggggaacac aaagcctctg cttactcctg 7140
   aaagagtgct gttttctgtc ctgtatgtgt gacgtgtctg tgagcgtgca agaagcccct 7200
   atcttgactg ggacaatggc cagtgagtgt agctggggaa gaattgagag catgtccagg 7260
   tcccttcccc agccaacgcc caagatcagg ccacagcctc ctcacaatca attgcctcct 7320
   cactccttga tcactcagtg ctgcccaggc ccagcagaac agactctgcc agcaggcccc 7380
   actagcccca gctcctcttt gggtctcagg tcccctgagg atatggggct tcacctgaaa 7440
   tggtctgagg gcttttcctt ctacacagca ggcatcaaga tcaccaaata aagggactat 7500
   tgtgcctgcc tggagccctg ccagaggttt gggcccagag gggcacacag caggtgctca 7560
   ataactgcat taaatgcact aacagtgagg aaacacgccc ctcagactaa gcagtgagtg 7620
   ctgctcacag aatagtcccc attgggggat ggcccaaaga gtcactttgg tccctctggg 7680
   aagtgagaag gcaagtgaga aggctgtgag tcttaacctc ctctagaggc ccacagacag 7740
   accattcatt tctaagtctc tacccagaga cgcactgtgc ttcccacctt ggcctgacat 7800
   gtggcagggt tagaacacac ctcctatccc ctgccagccc gcgttcatgc caagtagcac 7860
   atatatgcct aaactcagca cttccatagt gcagtgaata catgtgtgtg tacagcatct 7920
   ccgcatggat gtacaggatg tgtgtgtgtg tgcgtgcccc catgctgtct gcattcaggc 7980
   tgttcttttt ggtaagacag ctaaaaaaag aatggtctgt gaagggacac tccctagcac 8040
   gctgcaacac ctgaatatct ccttgaaagg agggatcttc tactgcagga gactcgtggt 8100
   aaaggtggcc aagaaacatg gcaacggtgg ggctgagggc aaatgctggg caactgtgct 8160
   tccccatgtt cccctccccg tagccaagac tcatttcatg gagggagatc tcagcttgga 8220
   agaaggcagg agtcactgag cctccccaat ccaaacccct gagaagtgtc ctccctctgg 8280
   cctcagaccc tgcatcctgt ggtcacagac ccacagtgag aaaggaccag gccctaagga 8340
   gctgtgctgt ctctccacgg cccagagcgg gggatgggga tggggatggg gatggggatg 8400
   gggatgggga tgggggtagg ggtgggggtg ctttggacta acgtggaggg aatggaaggc 8460
   aggcctggtt ccaccctgca tgcccgaccc tggccccagc agcccccaca aggagctcag 8520
   ctgaccctgg gtgtctccct gtgatgggaa ggggtaagac gaggactcaa aggcagaacc 8580
   tgcagagtgc cccagacgct gatacctgca cagtcagtgc cacccaccca ggagttgagg 8640
   aggcactggg ttttggggtg aggacactgg acacctccct gcttctttcc caggcagaca 8700
   atcctggcgc agctcccttg ggttgctgtg tctggtggag ctgatcacag gtgaggggca 8760
   gagggcagtc tggggtccgc ctatggccag aggagcaggt cagggcggcg ccttgccgcc 8820
   ccagctgtgg cctgtttgct caagacgctg aggtctcggg gccagctaac aattgttgag 8880
   caaaatcctt cgacaaactt cacctacgtg caaggactcg aggagggaat cactcttagg 8940
   agtgggagag taatgtcttt gcctgtgccc agtgaaggcc cattggagct gcagctcagc 9000
   taccactgtg tgggagagaa gctggaagac tgagggcttc ctgggctgct ggcccagggt 9060
   tgggagacag cagtcacctg gcttaccagg cctatgcctg aagccctggg aagccaggac 9120
   gcaggcccca ggctgggaca aagctaccct gaaggagggc aaaggctgcc aaggccaacc 9180
   ccatgcctgc caaggccagg cctggcccat ttggccaagg cctaaggtgt aaaacaaggg 9240
   gagaggtaca agaggctgtg gggtctggct gggatccttg gggtcttcct tctgcattct 9300
   ccaaacgcct agagccagca gaaacgtttc gtctgattag aagccatcat ttctatccca 9360
   atcccggaaa attgactgcg gtgcagagag ggaggcctga gaagcagccg taggggagaa 9420
   ggtccaagct aattaggagg cagcatccgg gggcccatta gagcgcaggc tgctgtcact 9480
   cagccgggct gagttcccgg gagaagaggc tggagaagga ggggcaggcg gcccctcgac    9540
   gaggacaccg ctgggagctg ccggaacggg ccccgggctc tgcccccgcc ccggcgctgg    9600
   ctcgaaggcg cccgctcggt gcgatcctgt tcggcaaaca ttcactcatc ctgggctgtt    9660
   ctcgccaggg ctggggactt cgaggcggcc gagacgggag ttgattctag gcgaaacaag    9720
   tcatttgagg cctgaggtgt gcacgagccg cccgggactc gcaggccaga tgcgtttctt    9780
   ttgtgaggcc gagggagaac tcggtgtgtc accggggaag gagggagagg cgcggcgagg    9840
   ccgcgggggg cggggaggcg gcgggaaggt ggctgcggag ggggagggcg cgggcgaggc    9900
   agggagggag ggagggcggc agtgagggcg cggcggcgcg ggcggcttgg ggctggattc    9960
   cgcccgcgct ccctcgctcg ctcgctccct ccccagcccc ctccca                 10006
<210> 2
   <211> 2373
   <212> DNA
   <213> HomoSapiens
<400> 2
   cccaggccca ccccacccag cacccctggc gcagggactg ctggaacctg gctgtgcgcg      60
   ctgtcgcttt aagacagact ctgccggcgc cgtccggagc cttagaaacc ggccccggat     120
   cgcgagccgg agccggagcc ggagccgggg ccggccgggc tgctgaggcc cgagcggcag     180
   gagcgcagcg cggagcgctg agccaggcgc ccagtcgcga gaagctgccg ccgcctctgc     240
   ccgcccggcg ccgcagcccc gggcggtcca tggggcgggc acggcgtcgc tgcaggcgcc     300
   ggcagccctg gagggcagcc gcttaggcgc tgcgctcttg tccccgcagg tcgcagccag     360
   ggcggcgggg cgcgcccagc cccggcccct ggagcgcccg ccgcggtccc cacctccatg     420
   gacgccttca aggggggcat gagcctggag cggctgccgg aggggctccg gccgccgccg     480
   ccgccacccc atgacatggg gcccgccttc cacctggccc ggcccgccga cccccgcgag     540
   ccgctcgaga actccgccag cgagtcgtct gacacggagc tgccagagaa ggagcgcggc     600
   ggggaaccca aggggcccga ggacagtggt gcgggaggca cgggctgcgg cggcgcagac     660
   gacccagcca agaagaagaa gcagcggcgg caacgtacgc acttcacaag ccagcagttg     720
   caagagctag aggccacgtt ccagaggaac cgctaccccg acatgagcat gagggaggag     780
   atcgccgtgt ggaccaacct caccgagccg cgcgtgcggg tctggttcaa gaaccggcga     840
   gccaagtggc gtaagcgcga gcgtaaccag cagctggacc tgtgcaaggg tggctacgtg     900
   ccgcagttca gcggcctagt gcagccctac gaggacgtgt acgccgccgg ctactcctac     960
   aacaactggg ccgccaagag cctggcgcca gcgccgctct ccaccaagag cttcaccttc 1020
   ttcaactcca tgagcccgct gtcgtcgcag tccatgttct cagcacccag ctccatctcc 1080
   tccatgacca tgccgtccag catgggccca ggcgccgtgc ctggcatgcc caactcgggc 1140
   ctcaacaaca tcaacaacct caccggctcc tcgctcaact cggccatgtc gccgggcgct 1200
   tgcccgtacg gcactcccgc ctcgccctac agcgtctacc gggacacgtg caactcgagc 1260
   ctagccagcc tgcggctcaa gtccaaacag cactcgtcgt ttggctacgg cgccctgcag 1320
   ggcccggcct cgggcctcaa cgcgtgccag tacaacagct gaccgccccg ccgcaccacg 1380
   cgggccggcg gccggagcgg ggaagggcgc gggcgcggag gacgcacgcg gggccccggc 1440
   tcgcaagccc cagctcaccg cgccgcggac ctcacacctg cgcagccccc tcctcccact 1500
   tcccactccg ggttggtttt gtgtttgctt ttccggaccc cactctgccc tccaaaaaga 1560
   caaaaaaaaa aaaaaaaaaa aaagcaaaaa gacgtcggag aaaagtgccg cgaaaaaatg 1620
   gatgagttgc aatttctctc gggatggcgc gggtggtgtg tgtgtgttcc cacgggcccc 1680
   ggaggcccac tccgcggagg gcacgcggcg cggtaggcga gcgccgaggc ccagcggccg 1740
   ggggaggacg acctcgtatc ccgcgtcccc gccgcgctgg atccggactg agtggccggg 1800
   cctgcggact ggatgtgcgg ggcctggact tgcctaggat ttcccgaccc cgtacaaacc 1860
   aagttgccct ctccgagcta ggcccggccg agagcgcctt agctcgagtc ggatccgtgt 1920
   tggggcgggc gttgggtttg gggggacggt gcccccagcc caggatcggg cactcagtgg 1980
   agccgcacac ggccccggcg cgcctggtag agcctcgctg gccccgcgcc ccggagccct 2040
   atattaaggc cacggagcga cagcgggcag tgcgggcctg gcgggaggtg ggggaggtcc 2100
   atctcagaac accccagcct tgagcttagc tgcaggccca ggccctctgc tctgctcccg 2160
   ggctaggagg tggccctctg tctgggcgaa cagccccctc ctcaccgccc gccgtgcaag 2220
   agtcgagccg gcagagcaag gggcgcggcc ccagggccct gcgcccactt tgcacacccg 2280
   ctctccggcc cgcgcccctg tttacagcgt ccctgtgtat gttggactga ctgtaataaa 2340
   tctgtctata tcgactaaaa aaaaaaaaaa aaa 2373
<210> 3
   <211> 314
   <212> PRT
   <213> HomoSapiens
<400> 3
   Met Asp Ala Phe Lys Gly Gly Met Ser Leu Glu Arg Leu Pro Glu Gly
   1               5                   10                  15
   Leu Arg Pro Pro Pro Pro Pro Pro His Asp Met Gly Pro Ala Phe His
               20                  25                  30
   Leu Ala Arg Pro Ala Asp Pro Arg Glu Pro Leu Glu Asn Ser Ala Ser
           35                  40                  45
   Glu Ser Ser Asp Thr Glu Leu Pro Glu Lys Glu Arg Gly Gly Glu Pro
       50                  55                  60
   Lys Gly Pro Glu Asp Ser Gly Ala Gly Gly Thr Gly Cys Gly Gly Ala
   65                  70                  75                  80
   Asp Asp Pro Ala Lys Lys Lys Lys Gln Arg Arg Gln Arg Thr His Phe
                   85                  90                  95
   Thr Ser Gln Gln Leu Gln Glu Leu Glu Ala Thr Phe Gln Arg Asn Arg
               100                 105                 110
   Tyr Pro Asp Met Ser Met Arg Glu Glu Ile Ala Val Trp Thr Asn Leu
           115                 120                 125
   Thr Glu Pro Arg Val Arg Val Trp Phe Lys Asn Arg Arg Ala Lys Trp
       130                 135                 140
   Arg Lys Arg Glu Arg Asn Gln Gln Leu Asp Leu Cys Lys Gly Gly Tyr
   145                 150                 155                 160
   Val Pro Gln Phe Ser Gly Leu Val Gln Pro Tyr Glu Asp Val Tyr Ala
                   165                 170                 175
   Ala Gly Tyr Ser Tyr Asn Asn Trp Ala Ala Lys Ser Leu Ala Pro Ala
               180                 185                 190
   Pro Leu Ser Thr Lys Ser Phe Thr Phe Phe Asn Ser Met Ser Pro Leu
           195                 200                 205
   Ser Ser Gln Ser Met Phe Ser Ala Pro Ser Ser Ile Ser Ser Met Thr
       210                 215                 220
   Met Pro Ser Ser Met Gly Pro Gly Ala Val Pro Gly Met Pro Asn Ser
   225                 230                 235                 240
   Gly Leu Asn Asn Ile Asn Asn Leu Thr Gly Ser Ser Leu Asn Ser Ala
                   245                 250                 255
   Met Ser Pro Gly Ala Cys Pro Tyr Gly Thr Pro Ala Ser Pro Tyr Ser
               260                 265                 270
   Val Tyr Arg Asp Thr Cys Asn Ser Ser Leu Ala Ser Leu Arg Leu Lys
           275                 280                 285
   Ser Lys Gln His Ser Ser Phe Gly Tyr Gly Ala Leu Gln Gly Pro Ala
       290                 295                 300
   Ser Gly Leu Asn Ala Cys Gln Tyr Asn Ser
   305                 310
<210> 4
   <211> 18
   <212> DNA
   <213> Artificial Sequence
<220>
   <223> primer
<400> 4
   ggaaatcgtg cgtgacat 18
<210> 5
   <211> 23
   <212> DNA
   <213> Artificial Sequence
<220>
   <223> primer
<400> 5
   tcatgatgga gttgaatgta gtt 23
<210> 6
   <211> 20
   <212> DNA
   <213> Artificial Sequence
<220>
   <223> primer
<400> 6
   gacccagcca agaagaagaa 20
<210> 7
   <211> 22
   <212> DNA
   <213> Artificial Sequence
<220>
   <223> primer
<400> 7
   gaggttgttg atgttgttga gg 22
<210> 8
   <211> 20
   <212> DNA
   <213> Artificial Sequence
<220>
   <223> primer
<400> 8
   ctgtttgctc aagacgctga 20
<210> 9
   <211> 20
   <212> DNA
   <213> Artificial Sequence
<220>
   <223> primer
<400> 9
   ctcggcctca caaaagaaac 20
<210> 10
   <211> 20
   <212> DNA
   <213> Artificial Sequence
<220>
   <223> primer
<400> 10
   tgtctgcatt caggctgttc 20
<210> 11
   <211> 20
   <212> DNA
   <213> Artificial Sequence
<220>
   <223> primer
<400> 11
   gattccctcc tcgagtcctt 20
<210> 12
   <211> 20
   <212> DNA
   <213> Artificial Sequence
<220>
   <223> primer
<400> 12
   caagtgagct ggatgctgaa 20
<210> 13
   <211> 20
   <212> DNA
   <213> Artificial Sequence
<220>
   <223> primer
<400> 13
   agggagtgtc ccttcacaga 20
<210> 14
   <211> 20
   <212> DNA
   <213> Artificial Sequence
<220>
   <223> primer
<400> 14
   gctcagccat tctcaggaac 20
<210> 15
   <211> 20
   <212> DNA
   <213> Artificial Sequence
<220>
   <223> primer
<400> 15
   gccattgtcc cagtcaagat 20
<210> 16
   <211> 20
   <212> DNA
   <213> Artificial Sequence
<220>
   <223> primer
<400> 16
   tcgcgtcaag agggtatttt 20
<210> 17
   <211> 20
   <212> DNA
   <213> Artificial Sequence
<220>
   <223> primer
<400> 17
   taggacccat ggctctaccc 20
<210> 18
   <211> 20
   <212> DNA
   <213> Artificial Sequence
<220>
   <223> primer
<400> 18
   cacgagtcag gtgggaaact 20
<210> 19
   <211> 20
   <212> DNA
   <213> Artificial Sequence
<220>
   <223> primer
<400> 19
   gacgtctgct gcttttctgc 20
<210> 20
   <211> 19
   <212> DNA
   <213> Artificial Sequence
<220>
   <223> primer
<400> 20
   aggcacggac tagcaggac 19
<210> 21
   <211> 18
   <212> DNA
   <213> Artificial Sequence
<220>
   <223> primer
<400> 21
   atgcggacga agccagag 18
<210> 22
   <211> 20
   <212> DNA
   <213> Artificial Sequence
<220>
   <223> primer
<400> 22
   ttagcattca gcccctctgt 20
<210> 23
   <211> 21
   <212> DNA
   <213> Artificial Sequence
<220>
   <223> primer
<400> 23
   ttcatgagat gcagtcagca g 21
<210> 24
   <211> 20
   <212> DNA
   <213> Artificial Sequence
<220>
   <223> primer
<400> 24
   acaactggta ggggcaacag 20
<210> 25
   <211> 20
   <212> DNA
   <213> Artificial Sequence
<220>
   <223> primer
<400> 25
   tgtgtggctt tggcaaataa 20
<210> 26
   <211> 20
   <212> DNA
   <213> Artificial Sequence
<220>
   <223> primer
<400> 26
   gcactgtgct ccaactgtgt 20
<210> 27
   <211> 20
   <212> DNA
   <213> Artificial Sequence
<220>
   <223> primer
<400> 27
   gggggagtgt tcttttcctt 20

## Description

### FIELD OF THE INVENTION

The present invention relates methods of stratifying adolescent idiopathic scoliosis, isolated nucleic acid molecules for use in same.

### BACKGROUND OF THE INVENTION

Spinal deformities and scoliosis in particular, represent the most prevalent type of orthopaedic deformities in children and adolescents (0.2-6% of the population). Published studies suggest that one percent to six percent of the population will develop scoliosis. This condition leads to the formation of severe deformities of the spine affecting mainly adolescent girls in number and severity.

At present, the cause of adolescent idiopathic scoliosis (AIS), remains unclear (Connor JM, Conner AN, Connor RA, Tolmie JL, Yeung B, Goudie D. Genetic aspects of early childhood scoliosis. Am J Med Genet. 1987;27:419-424; and Machida M. Cause of idiopathic scoliosis. Spine. 1999;24:2576-2583) and there remains a need to stratify children or adolescents having AIS, identify children or adolescents at risk of developing AIS and identify which of the affected individuals are at risk of progression.

It has been showed that Pitx1 +/- mice developed severe spinal deformities after weaning. Paired-like homeodomain transcription factor 1 (Pitx1, previously called Ptx1) is a homeodomain transcription factor detected initially throughout pituitary development. The Pitx-family contains three related members, Pitx1, Pitx2 and Pitx3, which are members of the paired class of homeodomain proteins. The three Pitx factors have similar transcription properties (Drouin,J., Lanctôt,C., & Tremblay,J.J. La famille Ptx des facteurs de transcription à homéodomaine. Médecine/Sciences 14, 335-339 (1998); Drouin,J., Lamolet,B., Lamonerie,T., Lanctot,C., & Tremblay,J.J. The PTX family of homeodomain transcription factors during pituitary developments. Mol. Cell Endocrinol. 140, 31-36 (1998); and Lanctôt,C., Lamolet,B., & Drouin,J. The bicoid-related homeoprotein Ptx1 defines the most anterior domain of the embryo and differentiates posterior from anterior lateral mesoderm. Development 124, 2807-2817 (1997)). Among others, this transcription factor controls the development of craniofacial and hind limb specific structures in mammals. The *pitx1* gene is highly expressed in mouse hind limb long bones during development and accumulation of high levels of Pitx1 proteins were detected by immunohistochemistry on hind limb long bone sections mainly in the periarticular region, along the perichondrium (including at the hip and knee joints) and also in the nuclei of proliferative chondrocytes (Lanctôt,C., Lamolet,B., & Drouin,J. The bicoid-related homeoprotein Ptx1 defines the most anterior domain of the embryo and differentiates posterior from anterior lateral mesoderm. Development 124, 2807-2817 (1997)). Pitx1 expression was also detected in craniofacial structures such as the mandible and at the temporo-mandibular joints. It has been shown that targeted inactivation of the mouse pitx1 gene severely impairs craniofacial and hind limb development. While null mice died at birth, all PITX1 +/- mice which are normal at birth, developed severe spinal deformities (100% starting at 2 months).

The present description refers to a number of documents.

### SUMMARY OF THE INVENTION

In accordance with the present invention, there is provided a method of stratifying a subject having adolescent idiopathic scoliosis (AIS) comprising: providing a cell sample isolated from the subject; detecting Paired-like homeodomain transcription factor 1 (Pitx1) expression in the cell sample; whereby the results of the detecting step enables the stratification of the subject having AIS as belonging to an AIS subclass.

In accordance with another aspect of the present invention, there is provided a method of stratifying a subject having adolescent idiopathic scoliosis (AIS) for a clinical trial comprising: providing a cell sample isolated from the subject; detecting Paired-like homeodomain transcription factor 1 (Pitx1) expression in the cell sample; and stratifying the subject for a clinical trial based on the results of the detecting step.

In accordance with another aspect of the present invention, there is provided a method for predicting a risk for developing adolescent idiopathic scoliosis (AIS) in a subject comprising providing a cell sample isolated from the subject; and detecting Paired-like homeodomain transcription factor 1 (Pitx1) expression in the cell sample; wherein an absence of Pitx1 expression is indicative that the subject is at risk for developing AIS.

In a specific embodiment of these methods, an absence of Pitx1 expression is indicative that the subject is at risk for developing a Cobb's angle of 45° and above. In another specific embodiment, the detecting step is performed with an isolated nucleic acid molecules specific to a Pitx1 transcription product. In another specific embodiment, the isolated nucleic acid molecule is detectably labeled. In another specific embodiment, the detecting step is performed with an antibody that binds specifically to Pitx1. In another specific embodiment, the cell sample is selected from the group consisting of an osteoblasts sample, a chondrocytes sample, a skeletal myoblasts sample and a blood sample. In another specific embodiment, the cell sample is an osteoblasts sample. In another specific embodiment, the method further comprises a step of selecting a preventive action or a treatment in light of the results of the detecting step. In another specific embodiment, said subject is pre-diagnosed as being a likely candidate for developing adolescent idiopathic scoliosis.

In accordance with another aspect of the present invention, there is provided a method of selecting a compound potentially useful in the treatment of adolescent idiopathic scoliosis, said method comprising the steps of (a) contacting a test compound with at least one cell known to express Paired-like homeodomain transcription factor 1 (Pitx1); and (b) determining Pitx1 expression level; wherein the test compound is selected if Pitx1 expression level is increased in the presence of the test compound as compared to that in the absence thereof.

In a specific embodiment, said cell is an osteoblast. In another specific embodiment, said cell is from a subject having adolescent idiopathic scoliosis (AIS). In another specific embodiment, the subject is a human.

Disclosed is a comprising an isolated nucleic acid molecule specific to a transcription product of a Paired-like homeodomain transcription factor 1 (Pitx1) and instructions to use the probe to predict whether a subject is at risk for developing adolescent idiopathic scoliosis.

Disclosed is a kit comprising an isolated nucleic acid molecule specific to a transcription product of a Paired-like homeodomain transcription factor 1 (Pitx1) and instructions to use the probe to stratify a subject having adolescent idiopathic scoliosis.

Further disclosed is the kit which further comprises a container for a nucleotide sample from the subject.

Further disclosed is a kit comprising an antibody specific to a Paired-like homeodomain transcription factor 1 (Pitx1) and instructions to use the antibody to predict whether a subject is at risk for developing adolescent idiopathic scoliosis.

Further disclosed is a kit comprising an antibody specific to a Paired-like homeodomain transcription factor 1 (Pitx1) and instructions to use the antibody to stratify a subject having adolescent idiopathic scoliosis.

The articles "a," "an" and "the" are used herein to refer to one or to more than one (i.e., to at least one) of the grammatical object of the article.

The term "including" and "comprising" are used herein to mean, and re used interchangeably with, the phrases "including but not limited to" and "comprising but not limited to".

The terms "such as" are used herein to mean, and is used interchangeably with, the phrase "such as but not limited to".

As used herein the terms "likely candidate for developing adolescent idiopathic scoliosis" include children of which a least one parent has adolescent idiopathic scoliosis. Among other factors, age (adolescence), gender and other family antecedent are factors that are known to contribute to the risk of developing a scoliosis and are used to a certain degree to assess the risk of developing AIS. In certain subjects, scoliosis develops rapidly over a short period of time to the point of requiring a corrective surgery (often when the deformity reaches a Cobb's angle ≥ 50°). Current courses of action available from the moment AIS is diagnosed (when scoliosis is apparent) include observation (when Cobb's angle is around 10-25°), orthopaedic devices (when Cobb's angle is around 25-30°), and surgery (over 45°). A more reliable determination of the risk of progression could enable to 1) select an appropriate diet to remove certain food products identified as contributors to scoliosis; 2) select the best therapeutic agent; and/or 3) select the least invasive available treatment such as postural exercises, orthopaedic device, or less invasive surgeries or surgeries without fusions (a surgery that does not fuse vertebra and preserves column mobility). The present invention encompasses selecting the most efficient and least invasive known preventive actions or treatments in view of the determined risk of developing AIS. The present invention also encompasses stratifying AIS patients with methods of the present invention.

As used herein, the terms "severe AIS" refers to a scoliosis characterized by Cobb's angle of 45° or more.

As used herein, the term "Pitx1 expression" is used to refer Pitx1 transcription and/or Pitx1 translation. In a more specific embodiment, Pitx1 expression refers to Pitx1 transcription.

As used herein the terms "risk of developing AIS" and "risk of progression of AIS" are used interchangeably and refer to a genetic or metabolic predisposition of a subject to develop a scoliosis (i.e. spinal deformity) and/or a more severe scoliosis at a future time.

As used herein the term "subject" is meant to refer to any mammal including human, mice, rat, dog, cat, pig, monkey, horse, etc. In a particular embodiment, it refers to a human. In an other particular embodiment, it refers to a horse and more specifically a racing horse.

As used herein the terms "blood sample" is whole blood and it is a cell sample in that it comprises peripheral blood mononuclear cells.

Without being so limited, cells where Pitx1 is known to be expressed include cells from muscles, bone and cartilages such as osteoblasts, chondrocytes and skeletal myoblasts.

The present invention also relates to methods for the determination of the level of expression of transcripts or translation product of a single gene such as pitx1. The present invention therefore encompasses any known method for such determination including real time PCR and competitive PCR, Northern blots, nuclease protection, plaque hybridization and slot blots.

The present invention also concerns isolated nucleic acid molecules including probes and primers to detect Pitx1. In specific embodiments, the isolated nucleic acid molecules have no more than 300, or no more than 200, or no more than 100, or no more than 90, or no more than 80, or no more than 70, or no more than 60, or no more than 50, or no more than 40 or no more than 30 nucleotides. In specific embodiments, the isolated nucleic acid molecules have at least 17, or at least 18, or at least 19, or at least 20, or at least 30, or at least 40 nucleotides. In other specific embodiments, the isolated nucleic acid molecules have at least 20 and no more than 300 nucleotides. In other specific embodiments, the isolated nucleic acid molecules have at least 20 and no more than 200 nucleotides. In other specific embodiments, the isolated nucleic acid molecules have at least 20 and no more than 100 nucleotides. In other specific embodiments, the isolated nucleic acid molecules have at least 20 and no more than 90 nucleotides. In other specific embodiments, the isolated nucleic acid molecules have at least 20 and no more than 80 nucleotides. In other specific embodiments, the isolated nucleic acid molecules have at least 20 and no more than 70 nucleotides. In other specific embodiments, the isolated nucleic acid molecules have at least 20 and no more than 60 nucleotides. In other specific embodiments, the isolated nucleic acid molecules have at least 20 and no more than 50 nucleotides. In other specific embodiments, the isolated nucleic acid molecules have at least 20 and no more than 40 nucleotides. In other specific embodiments, the isolated nucleic acid molecules have at least 17 and no more than 40 nucleotides. In other specific embodiments, the isolated nucleic acid molecules have at least 20 and no more than 30 nucleotides. In other specific embodiments, the isolated nucleic acid molecules have at least 17 and no more than 30 nucleotides. In other specific embodiments, the isolated nucleic acid molecules have at least 30 and no more than 300 nucleotides. In other specific embodiments, the isolated nucleic acid molecules have at least 30 and no more than 200 nucleotides. In other specific embodiments, the isolated nucleic acid molecules have at least 30 and no more than 100 nucleotides. In other specific embodiments, the isolated nucleic acid molecules have at least 30 and no more than 90 nucleotides. In other specific embodiments, the isolated nucleic acid molecules have at least 30 and no more than 80 nucleotides. In other specific embodiments, the isolated nucleic acid molecules have at least 30 and no more than 70 nucleotides. In other specific embodiments, the isolated nucleic acid molecules have at least 30 and no more than 60 nucleotides. In other specific embodiments, the isolated nucleic acid molecules have at least 30 and no more than 50 nucleotides. In other specific embodiments, the isolated nucleic acid molecules have at least 30 and no more than 40 nucleotides. It should be understood that in real-time PCR, primers also constitute probe without the traditional meaning of this term. Primers or probes appropriate to detect Pitx1 in the methods of the present invention can be designed with known methods using sequences distributed across the Pitx1 nucleotide sequence. (Buck et al. Design Strategies and Performance of Custom DNA Sequencing primers. Biotechniques 27:528-536 (September 1999)).

Although amino acid and nucleotide sequences for Pitx1 are included herein, the present invention is not so limited and encompasses the detection of any Pitx1 protein or nucleotides isolated from a subject. Without being so limited, the present invention encompasses the detection of the Pitx1 presented in Table 1.

**TABLE 1. ACCESSION NUMBERS FOR PITX1 SEQUENCES**

| **Nucleotide** | | **Protein** |
|---|---|---|
| Genomic | AC004764.1 | AAC17733.1 |
| Genomic | AC008406.7 (17530..24049, complement) | None |
| Genomic | AF009648.1 | AAB65251.1 |
| Genomic | AF009649.1 | AAB65251.1 |
| Genomic | AF009650.1 | AAB65251.1 |
| Genomic | CH471062.2 | EAW62226.1 |
| | | EAW62227.1 |
| Genomic | CS278249.1 | CAJ86537.1 |
| mRNA | AK290635.1 | BAF83324.1 |
| mRNA | AL578756.2 | None |
| mRNA | BC003685.1 | AAH03685.1 |
| mRNA | BC009412.1 | AAH09412.1 |
| mRNA | BX362641.2 | None |
| mRNA | CR601326.1 | None |
| mRNA | CR603120.1 | None |
| mRNA | CR610821.1 | None |
| mRNA | U70370.1 | AAC51126.1 |
| Synthetic | EU446647.1 | ABZ92176.1 |
| | | P78337.2 |
| mRNA | NM_002653 version NM_002653.4 | NP_002644 version NP_002644.4 |
| Chromosome 5, reference assembly, complete sequence | NC_000005(134391323-134397863) | NC_000005.8 |
| Genomic | NC_000005.8 Reference assembly 134397863..134391323, complement | |
| Genomic | NT_034772.5 36784977..36778437, complement | |
| Genomic | AC_000048.1 130493751..130487209, complement | |
| Genomic | NW_922784.1 8121767..8115225, complement | |
| Genomic | AC_000137.1 91265592..91277386 | |
| Genomic | NW_001838952.2 4413502..4425296 | |
| Genomic | NW_001838952.2 4413502..4425296 | |

Probes of the invention can be utilized with naturally occurring sugar-phosphate backbones as well as modified backbones including phosphorothioates, dithionates, alkyl phosphonates and α-nucleotides and the like. Modified sugar-phosphate backbones are generally known (Miller, 1988. Ann. Reports Med. Chem. 23:295; Moran et al., 1987. Nucleic Acids Res., 14:5019.). Probes of the invention can be constructed of either ribonucleic acid (RNA) or deoxyribonucleic acid (DNA), and preferably of DNA.

The types of detection methods in which probes can be used include Southern blots (DNA detection), dot or slot blots (DNA, RNA), and Northern blots (RNA detection). Although less preferred, labeled proteins could also be used to detect a particular nucleic acid sequence to which it binds. Other detection methods include kits containing probes on a dipstick setup and the like.

As used herein the terms "detectably labeled" refer to a marking of a probe or antibody in accordance with the presence invention that will allow the detection of the Pitx1 expression in methods and kits of the present invention. Although the present invention is not specifically dependent on the use of a label for the detection of a particular nucleic acid sequence, such a label might be beneficial, by increasing the sensitivity of the detection. Furthermore, it enables automation. Probes can be labeled according to numerous well known methods (Sambrook, J., Fritsch, E. F. & Maniatis, T. (1989). Molecular Cloning: A Laboratory Manual, Cold Spring Harbor Laboratory Press, Cold Spring Harbor, NY). Non-limiting examples of labels include 3H, 14C, 32P, and 35S. Non-limiting examples of detectable markers include ligands, fluorophores, chemiluminescent agents, enzymes, and antibodies. Other detectable markers for use with probes, which can enable an increase in sensitivity of the method of the invention, include biotin and radionucleotides. It will become evident to the person of ordinary skill that the choice of a particular label dictates the manner in which it is bound to the probe or antibody.

As commonly known, radioactive nucleotides can be incorporated into probes of the invention by several methods. Non-limiting examples thereof include kinasing the 5' ends of the probes using gamma 32P ATP and polynucleotide kinase, using the Klenow fragment of Pol I of E. coli in the presence of radioactive dNTP (e.g. uniformly labeled DNA probe using random oligonucleotide primers in low-melt gels), using the SP6/T7 system to transcribe a DNA segment in the presence of one or more radioactive NTP, and the like.

The present invention also relates to methods of selecting compounds. As used herein the term "compound" is meant to encompass natural, synthetic or semi-synthetic compounds, including without being so limited chemicals, macromolecules, cell or tissue extracts (from plants or animals), nucleic acid molecules, peptides, antibodies and proteins.

The present invention also relates to arrays. As used herein, an "array" is an intentionally created collection of molecules which can be prepared either synthetically or biosynthetically. The molecules in the array can be identical or different from each other. The array can assume a variety of formats, e.g., libraries of soluble molecules; libraries of compounds tethered to resin beads, silica chips, or other solid supports.

As used herein "array of nucleic acid molecules" is an intentionally created collection of nucleic acids which can be prepared either synthetically or biosynthetically in a variety of different formats (e.g., libraries of soluble molecules; and libraries of oligonucleotides tethered to resin beads, silica chips, or other solid supports). Additionally, the term "array" is meant to include those libraries of nucleic acids which can be prepared by spotting nucleic acids of essentially any length (e.g., from 1 to about 1000 nucleotide monomers in length) onto a substrate. The term "nucleic acid" as used herein refers to a polymeric form of nucleotides of any length, either ribonucleotides, deoxyribonucleotides or peptide nucleic acids (PNAs), that comprise purine and pyrimidine bases, or other natural, chemically or biochemically modified, non-natural, or derivatized nucleotide bases. The backbone of the polynucleotide can comprise sugars and phosphate groups, as may typically be found in RNA or DNA, or modified or substituted sugar or phosphate groups. A polynucleotide may comprise modified nucleotides, such as methylated nucleotides and nucleotide analogs. The sequence of nucleotides may be interrupted by non-nucleotide components. Thus the terms nucleoside, nucleotide, deoxynucleoside and deoxynucleotide generally include analogs such as those described herein. These analogs are those molecules having some structural features in common with a naturally occurring nucleoside or nucleotide such that when incorporated into a nucleic acid or oligonucleotide sequence, they allow hybridization with a naturally occurring nucleic acid sequence in solution. Typically, these analogs are derived from naturally occurring nucleosides and nucleotides by replacing and/or modifying the base, the ribose or the phosphodiester moiety. The changes can be tailor made to stabilize or destabilize hybrid formation or enhance the specificity of hybridization with a complementary nucleic acid sequence as desired.

As used herein "solid support", "support", and "substrate" are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations.

Any known nucleic acid arrays can be used in accordance with the present invention. For instance, such arrays include those based on short or longer oligonucleotide probes or primers as well as cDNAs or polymerase chain reaction (PCR) products (Lyons P., 2003. Advances in spotted microarray ressources for expression profiling. Briefings in Functionnal Genomics and Proteomics 2, 21-30). Other methods include serial analysis of gene expression (SAGE), differential display, (Ding G. and Cantor C.R., 2004. Quantitative analysis of nucleic acids - the last few years of progress. J Biochem Biol 37, 1-10) as well as subtractive hybridization methods (Scheel J., Von Brevern M.C., Horlein A., Fisher A., Schneider A., Bach A. 2002. Yellow pages to the transcriptome. Pharmacogenomics 3, 791-807), differential screening (DS), RNA arbitrarily primer (RAP)-PCR, restriction endonucleolytic analysis of differentially expressed sequences (READS), amplified restriction fragment-length polymorphisms (AFLP).

"Stringent hybridization conditions" and "stringent hybridization wash conditions" in the context of nucleic acid hybridization experiments such as Southern and Northern hybridization are sequence dependent, and are different under different environmental parameters. The Tₘ is the temperature (under defined ionic strength and pH) at which 50% of the target sequence hybridizes to a perfectly matched probe. Specificity is typically the function of post-hybridization washes, the critical factors being the ionic strength and temperature of the final wash solution. For DNA-DNA hybrids, the Tₘ can be approximated from the equation of Meinkoth and Wahl, 1984; Tₘ 81.5°C + 16.6 (log M) +0.41 (%GC)-0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. Tₘ is reduced by about 1°C for each 1% of mismatching; thus, Tₘ, hybridization, and/or wash conditions can be adjusted to hybridize to sequences of the desired identity. For example, if sequences with >90% identity are sought, the Tₘ can be decreased 10°C. Generally, stringent conditions are selected to be about 5°C lower than the thermal melting point I for the specific sequence and its complement at a defined ionic strength and pH. However, severely stringent conditions can utilize a hybridization and/or wash at 1, 2, 3, or 4°C lower than the thermal melting point I; moderately stringent conditions can utilize a hybridization and/or wash at 6, 7, 8, 9, or 10°C lower than the thermal melting point I; low stringency conditions can utilize a hybridization and/or wash at 11, 12, 13, 14, 15, or 20°C lower than the thermal melting point I. Using the equation, hybridization and wash compositions, and desired T, those of ordinary skill will understand that variations in the stringency of hybridization and/or wash solutions are inherently described. If the desired degree of mismatching results in a T of less than 45°C (aqueous solution) or 32°C (formamide solution), it is preferred to increase the SSC concentration so that a higher temperature can be used. An extensive guide to the hybridization of nucleic acids is found in Tijssen, 1993. Generally, highly stringent hybridization and wash conditions are selected to be about 5°C lower than the thermal melting point Tₘ for the specific sequence at a defined ionic strength and pH.

An example of highly stringent wash conditions is 0.15 M NaCl at 72°C for about 15 minutes. An example of stringent wash conditions is a 0.2X SSC wash at 65°C for 15 minutes (see Sambrook, J., Fritsch, E. F. & Maniatis, T. (1989). Molecular Cloning: A Laboratory Manual, Cold Spring Harbor Laboratory Press, Cold Spring Harbor, NY for a description of SSC buffer). Often, a high stringency wash is preceded by a low stringency wash to remove background probe signal. An example medium stringency wash for a duplex of, e.g., more than 100 nucleotides, is 1X SSC at 45°C for 15 minutes. An example low stringency wash for a duplex of, e.g., more than 100 nucleotides, is 4-6X SSC at 40°C for 15 minutes. For short probes (e.g., about 10 to 50 nucleotides), stringent conditions typically involve salt concentrations of less than about 1.5 M, more preferably about 0.01 to 1.0 M, Na ion concentration (or other salts) at pH 7.0 to 8.3, and the temperature is typically at least about 30°C and at least about 60°C for long robes (e.g., >50 nucleotides). Stringent conditions may also be achieved with the addition of destabilizing agents such as formamide. In general, a signal to noise ratio of 2X (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code.

Very stringent conditions are selected to be equal to the Tₘ for a particular probe. An example of stringent conditions for hybridization of complementary nucleic acids which have more than 100 complementary residues on a filter in a Southern or Northern blot is 50% formamide, e.g., hybridization in 50% formamide, 1 M NaCl, 1% SDS at 37°C, and a wash in 0. 1 X SSC at 60 to 65°C. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C.

Washing with a solution containing tetramethylammonium chloride (TeMAC) could allow the detection of a single mismatch using oligonucleotide hybridyzation since such mismatch could generate a 10°C difference in the annealing temperature. The formulation to determine the washing temperature is Tm (°C) =] -682 (L⁻¹) + 97 where L represents the length of the oligonucleotide that will be used for the hybridization.

Disclosed is a kit for stratifying AIS and/or predicting whether a subject is at risk of developing AIS comprising an isolated nucleic acid, a protein or a ligand such as an antibody.
For example, a compartmentalized kit includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the subject sample (DNA genomic nucleic acid, cell sample or blood samples), a container which contains in some kits of the present invention, the probes used in the methods of the present invention, containers which contain enzymes, containers which contain wash reagents, and containers which contain the reagents used to detect the extension products. Kits may also contain instructions to use these probes and or antibodies to stratify AIS or predict whether a subject is at risk of developing AIS.

As used herein, the term "purified" in the expression "purified antibody" is simply meant to distinguish man-made antibody from an antibody that may naturally be produced by an animal against its own antigens. Hence, raw serum and hybridoma culture medium containing anti-Pitx1 antibody are "purified antibodies" within the meaning of the present invention.

As used herein, the term "ligand" broadly refers to natural, synthetic or semi-synthetic molecules. The term "molecule" therefore denotes for example chemicals, macromolecules, cell or tissue extracts (from plants or animals) and the like. Non limiting examples of molecules include nucleic acid molecules, peptides, antibodies, carbohydrates and pharmaceutical agents. The ligand appropriate for the present invention can be selected and screened by a variety of means including random screening, rational selection and by rational design using for example protein or ligand modeling methods such as computer modeling. The terms "rationally selected" or "rationally designed" are meant to define compounds which have been chosen based on the configuration of interacting domains of the present invention. As will be understood by the person of ordinary skill, macromolecules having non-naturally occurring modifications are also within the scope of the term "ligand". For example, peptidomimetics, well known in the pharmaceutical industry and generally referred to as peptide analogs can be generated by modeling as mentioned above.

### Antibodies

Both monoclonal and polyclonal antibodies directed to Pitx1 are included within the scope of this invention as they can be produced by well established procedures known to those of skill in the art. Additionally, any secondary antibodies, either monoclonal or polyclonal, directed to the first antibodies would also be included within the scope of this invention.

As used herein, the term "anti-Pitx1 antibody" or "immunologically specific anti- Pitx1 antibody" refers to an antibody that specifically binds to (interacts with) a Pitx1 protein and displays no substantial binding to other naturally occurring proteins other than the ones sharing the same antigenic determinants as the Pitx1 protein. The term antibody or immunoglobulin is used in the broadest sense, and covers monoclonal antibodies (including full length monoclonal antibodies), polyclonal antibodies, multispecific antibodies, and antibody fragments so long as they exhibit the desired biological activity. Antibody fragments comprise a portion of a full length antibody, generally an antigen binding or variable region thereof. Examples of antibody fragments include Fab, Fab', F(ab')₂, and Fv fragments, diabodies, linear antibodies, single-chain antibody molecules, single domain antibodies (e.g., from camelids), shark NAR single domain antibodies, and multispecific antibodies formed from antibody fragments. Antibody fragments can also refer to binding moieties comprising CDRs or antigen binding domains including, but not limited to, VH regions (V_{H}, V_{H}-V_{H}), anticalins, PepBodies™, antibody-T-cell epitope fusions (Troybodies) or Peptibodies. Additionally, any secondary antibodies, either monoclonal or polyclonal, directed to the first antibodies would also be included within the scope of this invention.

In general, techniques for preparing antibodies (including monoclonal antibodies and hybridomas) and for detecting antigens using antibodies are well known in the art (Campbell, 1984, In "Monoclonal Antibody Technology: Laboratory Techniques in Biochemistry and Molecular Biology", Elsevier Science Publisher, Amsterdam, The Netherlands) and in Harlow et al., 1988 (in: Antibody A Laboratory Manual, CSH Laboratories). The term antibody encompasses herein polyclonal, monoclonal antibodies and antibody variants such as single-chain antibodies, humanized antibodies, chimeric antibodies and immunologically active fragments of antibodies (e.g. Fab and Fab' fragments) which inhibit or neutralize their respective interaction domains in Hyphen and/or are specific thereto.

Polyclonal antibodies are preferably raised in animals by multiple subcutaneous (sc), intravenous (iv) or intraperitoneal (ip) injections of the relevant antigen with or without an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals may be immunized against the antigen, immunogenic conjugates, or derivatives by combining the antigen or conjugate (e.g., 100 µg for rabbits or 5 µg for mice) with 3 volumes of Freund's complete adjuvant and injecting the solution intradermally at multiple sites. One month later the animals are boosted with the antigen or conjugate (*e.g.*, with 1/5 to 1/10 of the original amount used to immunize) in Freund's complete adjuvant by subcutaneous injection at multiple sites. Seven to 14 days later the animals are bled and the serum is assayed for antibody titer. Animals are boosted until the titer plateaus. Preferably, for conjugate immunizations, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibodies may be made using the hybridoma method first described by Kohler et al., Nature, 256: 495 (1975), or may be made by recombinant DNA methods (e.g., U.S. Patent No. 6,204,023). Monoclonal antibodies may also be made using the techniques described in U.S. Patent Nos. 6,025,155 and 6,077,677 as well as U.S. Patent Application Publication Nos. 2002/0160970 and 2003/0083293 (see also, e.g., Lindenbaum et al., 2004).

In the hybridoma method, a mouse or other appropriate host animal, such as a rat, hamster or monkey, is immunized (e.g., as hereinabove described) to elicit lymphocytes that produce or are capable of producing antibodies that will specifically bind to the antigen used for immunization. Alternatively, lymphocytes may be immunized *in vitro.* Lymphocytes then are fused with myeloma cells using a suitable fusing agent, such as polyethylene glycol, to form a hybridoma cell.

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Other objects, advantages and features of the present invention will become more apparent upon reading of the following non-restrictive description of specific embodiments thereof, given by way of example only with reference to the accompanying drawings.

### BRIEF DESCRIPTION OF THE DRAWINGS

In the appended drawings:

Figure 1 compares Pitx1 expression in osteoblasts from severely affected AIS patients and matched control subjects. Reverse transcription-polymerase chain reaction for *pitx1* gene expression in human osteoblasts of control subjects (n=46) and patients with AIS (n=29). Pitx1 specific mRNA transcripts were detected in the control tissue. Loss of the *pitx1* gene expression was observed in all examined AIS samples and β-actin expression was used as internal control;

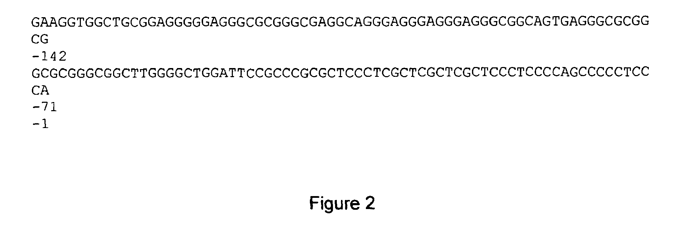

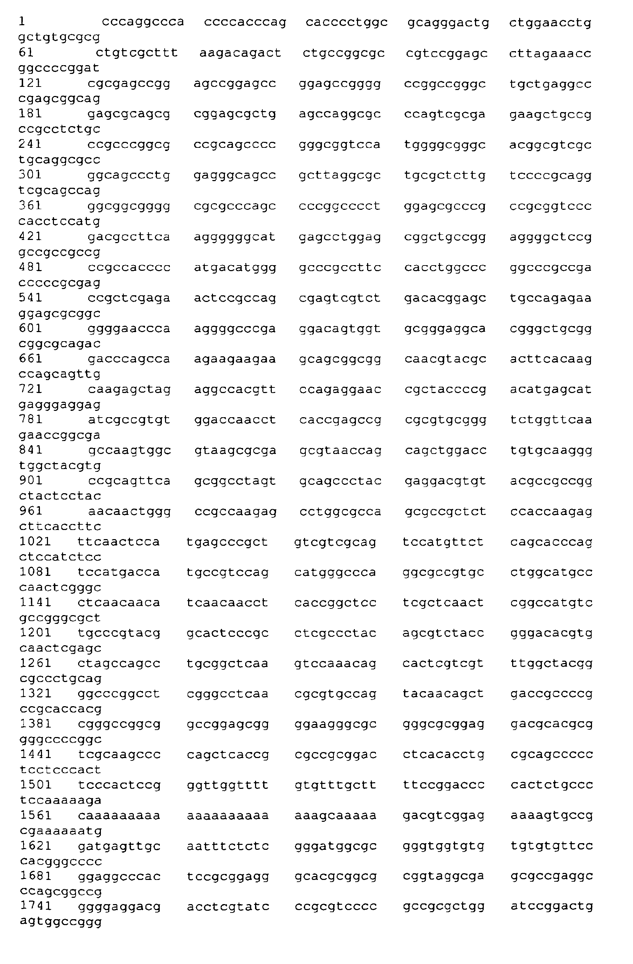

Figure 2 shows the sequence of a 10 kb pitx1 promoter region (SEQ ID NO: 1) and polymorphisms in that pitx1 promoter region between human subjects. The primers used to cover the different amplicons covering the 10 kb regions are provided in Table 4 below;

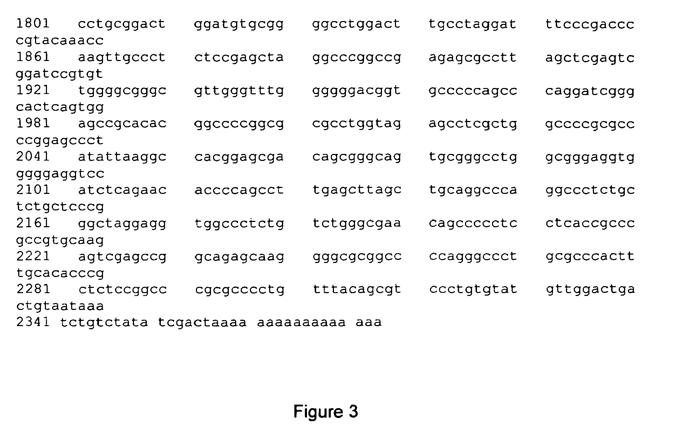

Figure 3 shows the sequence of the Pitx1 mRNA (SEQ ID NO: 2) (NM_002653); and

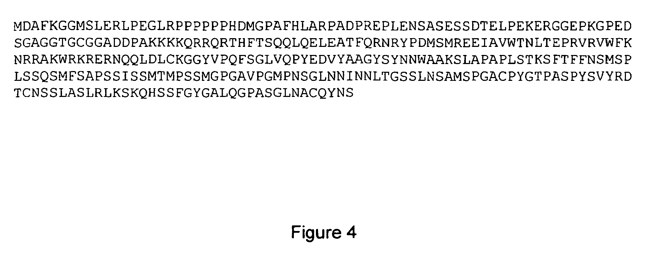

Figure 4 shows the Pitx1 amino acid sequence (SEQ ID NO: 3) (NP_002644).

### DESCRIPTION OF ILLUSTRATIVE EMBODIMENTS

The present invention demonstrated by RT-PCR analysis a loss of pitx1 gene expression in osteoblasts derived from biopsies obtained intraoperatively of severely affected AIS patients (n= 46) while osteoblasts derived from non-scoliotic patients (trauma cases) still expressed Pitx1 (n= 29).

The present invention is illustrated in further details by the following non-limiting examples.

### Human Specimens

Informed consent was obtained from all study participants as approved by each individual and collective Institutional Review Board (Ste-Justine University Hospital, Montreal's Children Hospital and The Shriners Hospital for Children all located in Montreal). All individuals were screened through a series of steps including history and clinical data, assuring the idiopathic nature of the problem. This was followed by a review of spinal radiographs. A person was deemed to be affected by AIS if history and physical examination were consistent with the diagnosis of idiopathic scoliosis and a minimum of a ten degree curvature in the coronal plane with vertebral rotation was found on the radiograph. Participants will also be screened as to the potential familial distribution of the disorder on a case by case basis. In the event that the disorder is found to be familial, family members will undergo parallel screening for the presence or absence of the condition. Other patients (without scoliosis) visiting our trauma clinics were used as controls.

The clinical characteristics of the examined AIS and control subjects are shown in Table 2 and Table 3 respectively. Scoliotic patients with a diagnostic other than AIS were tested as control subjects.

**TABLE 2. CLINICAL CHARACTERISTIC OF EXAMINED AIS SUBJECTS**

| **Number** | **GeN/Aer** | **Age** | **Curve type** | **Cobb angle** | **Heredity** |
|---|---|---|---|---|---|
| 1006 | f | 12,65 | double major | 61-46 | No |
| 1007 | m | 18,67 | right thoracolumbar | 61 | Yes |
| 1045 | f | 19,48 | left thoracolumbar | 38 | No |
| 1066 | f | 17,33 | right thoracic | 53 | Yes |
| 1137 | f | 20,54 | double major | 65-42 | No |
| 1167 | m | 14,58 | left thoracic | 49 | No |
| 1263 | f | 13,32 | double major | 53 | No |
| 1266 | m | 15,56 | double major | 52 | No |
| 1274 | f | 13,27 | double major | 42 | No |
| 1276 | f | 15,26 | left thoracic | 42 | No |
| 1277 | f | 12,73 | double major | 57-48 | No |
| 1280 | f | 14,4 | double major | 56-46 | No |
| 1294 | f | 16,92 | left thoracic | N/A | Yes |
| 1306 | f | 13,15 | double major | 77-48 | Yes |
| 1308 | f | 15,3 | double major | 77-20 | No |
| 1310 | f | 15,52 | double major | 55-42 | No |
| 1311 | f | 14,6 | double major | 78 | Yes |
| 1315 | f | 14,62 | right thoracic | 91 | No |
| 1317 | f | 13,97 | right thoracic | 53 | Yes |
| 1318 | f | 13,7 | left thoracolumbar | 49 | No |
| 1322 | f | 13,11 | double major | 51 | No |
| 1325 | f | 16,16 | left thoracic | 44 | Yes |
| 1329 | m | 14,02 | right thoracolumbar | 61 | Yes |
| 1335 | f | 17,6 | double major | 47-50 | No |
| 1337 | f | 14,13 | double major | 57-48 | Yes |
| 1339 | f | 14,28 | right thoracic | 31 | No |
| 1346 | f | 13,54 | double major | 50-34 | Yes |
| 1347 | f | 18,55 | double major | 56-45 | No |
| 1349 | f | 11,69 | left thoracolumbar | 74 | No |
| 1352 | f | 7,8 | right thoracic | 51 | No |
| 1360 | f | 9,92 | double major | 53-46 | Yes |
| 1385 | f | 16,06 | double major | 42-23 | No |
| 1390 | f | 15,61 | left thoracolumbar | 53 | No |
| 1391 | f | 15,01 | left lumbar | 54 | No |
| 1395 | f | 17,79 | left thoracolumbar | 84 | Yes |
| 1402 | f | 15,82 | right thoracic | 51 | No |
| 1406 | f | 14,89 | double major | 62-60 | No |
| 1409 | f | 13,62 | right thoracic | 40 | No |
| 1410 | f | 13,73 | right thoracic | 56 | Yes |
| 1417 | f | 13,24 | right thoracic | 59 | Yes |
| 1418 | f | 13,08 | right thoracic | 41 | No |
| 1420 | f | 13,42 | double major | 60-48 | Yes |
| 1422 | f | 12,44 | double major | 60-50 | Yes |
| 1425 | f | 13,42 | right thoracic | 68 | Yes |
| 1439 | f | N/A | right thoracic | 69 | Yes |
| 1442 | f | N/A | right thoracic | 60 | No |

| | | | | | |
|---|---|---|---|---|---|
| N/A : not available | | | | | |

**TABLE 3- CLINICAL CHARACTERISTICS OF EXAMINED CONTROL SUBJECTS**

| **Number** | **Gender** | **Age** | **Health status** | **Curve type** | **Cobb angle** | **Heredity** |
|---|---|---|---|---|---|---|
| C100 | f | N/A | healthy | nd | nd | No |
| C101 | N/A | N/A | healthy | nd | nd | No |
| C102 | f | 18 | healthy | nd | nd | No |
| C103 | N/A | N/A | healthy | nd | nd | No |
| C104 | f | 14 | healthy | nd | nd | No |
| C105 | f | 11 | healthy | nd | nd | No |
| C106 | m | 12 | healthy | nd | nd | No |
| C107 | f | 13 | healthy | nd | nd | No |
| C108 | f | 12 | healthy | nd | nd | No |
| C109 | m | 14 | healthy | nd | nd | No |
| C110 | m | 15 | healthy | nd | nd | No |
| C111 | m | 14 | healthy | nd | nd | No |
| C112 | f | 11 | healthy | nd | nd | No |
| C113 | m | 14 | healthy | nd | nd | No |
| C114 | N/A | N/A | healthy | nd | nd | No |
| C115 | m | 17 | healthy | nd | nd | No |
| C116 | m | 12 | healthy | and | nd | No |
| C117 | m | 16 | healthy | nd | nd | No |
| C118 | m | 12 | healthy | nd | nd | No |
| C119 | f | 15 | healthy | nd | nd | No |
| C120 | f | 15 | healthy | nd | nd | No |
| C121 | f | 8 | healthy | nd | nd | No |
| 1285 | f | 15,87 | paralytic scoliosis | N/A | 72 | Yes |
| 1293 | m | 11,79 | congenital scoliosis | left lumbar | 38 | No |
| 1341 | f | 11,14 | conqenital scoliosis | double major | 61-65 | No |
| 1375 | f | 13,71 | congenital scoliosis | right thoracolumbar | 53 | Yes |
| 1431 | m | 19,13 | neurological scoliosis | double major | 90-90 | No |
| 1434 | f | 12,43 | congenital scoliosis | double major | 79-77 | No |
| 1436 | f | 13,93 | kyphoscoliosis | kyphosis | 120 | No |

| | | | | | | |
|---|---|---|---|---|---|---|
| N/A: not available and nd: not detected | | | | | | |

### Osteoblasts cultures

Osteoblasts were obtained from bone specimens taken intraoperatively during spine surgeries of AIS patients and trauma surgeries in the case of control subjects. This cell type was chosen as cellular model for this study as described previously (Rodriguez MM, Ron D, Touhara K, Chen CH, Mochly-Rosen D. RACK1, a protein kinase C anchoring protein, coordinates the binding of activated protein kinase C and select pleckstrin homology domains in vitro. Biochemistry. 1999;38:13787-13794).

### Isolation of human osteoblasts

In all cases, osteoblasts were obtained intraoperatively from bone specimens originating from vertebras (varying from T3 to L4 according to the surgical procedure performed). Bony fragments were mechanically reduced to smaller pieces with a bone cutter in sterile conditions and incubated at 37°C in 5% CO₂ in a 100 mm culture dish in presence of DMEM medium containing 10% fetal bovine serum (FBS) (certified FBS, Invitrogen, Burlington, ON, Canada) and 1% penicillin/streptomycin (Invitrogen). After a 30-day period, the osteoblasts derived from the bone pieces were separated at confluence from the remaining bone fragments by trypsinization.

### Total RNA isolation and RT-PCR

Extraction of RNA from osteoblasts was done using the standard Trisol Reagent method. (Invitrogen).

The RNA obtained from the osteoblasts was used for cDNA synthesis performed with the Invitrogen Thermoscript™ RT-PCR system and the respective protocol in the following conditions: Enzyme used: *Taq* DNA polymerase from Invitrogen™. PCR conditions: 95°C 5minutes, Hot start (1 cycle). Following three reactions (32 cycles) : 94°C, 45 Seconds Denaturation; 55°C 45 Seconds; Primer annealing; 72°C 1 minute Elongation; 72°C 2 minutes Last elongation (1 cycle); 4°C 20 minutes pause; Duration: 2 hours 42 minutes. The quality of the cDNA was tested by amplifying 233bp fragment of human beta-actin using the sense primer 5'-GGAAATCGTGCGTGACAT-3' (SEQ ID NO: 4) and antisense primer 5'-TCATGATGGAGTTGAATGTAGTT-3' (SEQ ID NO: 5). For quantitative analysis, all amplifications were normalized against that of the housekeeping gene β-actin. PCR amplified product were separated on 1.5% agarose gel and visualized by ethidium bromide staining.

### Expression Analysis of Pitx1:

Coding region of Pitx1 501bp in length was amplified from the cDNA using the sense primer 5'- GACCCAGCCAAGAAGAAGAA-3' (SEQ 10 NO: 6) and the antisense primer 5'- GAGGTTGTTGATGTTGTTGAGG-3' (SEQ ID NO: 7) under the following PCR conditions: Enzyme used: Taq DNA polymerase from Invitrogen™. PCR conditions: 95°C 10 minutes hot start (1 cycle); Following three reactions (34 cycles): 94°C 45 Seconds Denaturation; 69°C 45 Seconds Primer annealing; 72°C 1 minute Elongation; 72°C 2 minutes Last elongation (1 cycle); 4°C 20 minutes; 4°C Pause; Duration: 2hours 34 minutes 11 seconds.

### Pitx1 promoter sequencing

10 kb region of the pitx1 promoter was amplified and sequenced to screen for mutations. Enzyme used: Platinum® Taq DNA polymerase High fidelity from Invitrogen™ under the following conditions:95°C 5 minutes hot start (1 cycle). Following three reactions 35 cycles: 94°C 45 Seconds Denaturation; 61.8°C 45 Seconds Primer annealing; 72°C 1 minute Elongation; 72°C 5 minutes Last elongation (1 cycle); 4°C 20 minutes; and 4°C Pause.

One hundred (100) ng of genomic DNA was mixed in a final volume of 25 µl containing 200 micromolar dNTPs, 1,5 mM MgCl₂, 10 pM of each primer (see Table 4 below for list of primers used), and 1 U Pfx DNA-polymerase (Invitrogen) or an other DNA polymerase.

PCR conditions: Regions PP1, PP2, PP3, PP6, PP7 were amplified using Platinum® pfx DNA polymerase from Invitrogen™ under the following PCR conditions: 95°C 5 minutes hot start (1 cycle); Following three reactions (35 cycles): 94°C 30 Seconds Denaturation; 60°C 30 Seconds; Primer annealing; 68°C 1 min 20 Sec Elongation; 68°C 2 minutes; Last elongation (1 cycle); 4°C 20 minutes; 4°C Pause.Duration : 2 hours 35 minutes 26 seconds

Regions PP4, PP5, PP8, PP9 and PP10 were amplified using Platinum®Taq DNA polymerase High fidelity from Invitrogen™ under the following conditions. 95°C 2 minutes hot start (1 cycle); Following three reactions (35 cycles); 94°C 45 Seconds Denaturation; 60°C 45 Seconds Primer annealing; 72°C 1 min 20 Sec Elongation; 72°C 5 minutes Last elongation (1 cycle); 4°C 20 minutes; 4°C Pause; Duration : 2 hours 53 minutes 4 seconds.

**TABLE 4: PITX PROMOTER PRIMERS**

| | |
|---|---|
| PP1 (962 bp) | forward primer 5'-CTGTTTGCTCAAGACGCTGA-3'; (SEQ ID NO: 8) |
| | reverse primer 5'-CTCGGCCTCACAAAAGAAAC-3' (SEQ ID NO: 9) |
| PP2 (966 bp) | forward primer 5'-TGTCTGCATTCAGGCTGTTC-3'; (SEQ ID NO: 10) |
| | reverse primer 5'-GATTCCCTCCTCGAGTCCTT-3' (SEQ ID NO: 11) |
| PP3 (1039 bp) | forward primer 5'-CAAGTGAGCTGGATGCTGAA-3'; (SEQ ID NO: 12) |
| | reverse primer 5'-AGGGAGTGTCCCTTCACAGA-3'(SEQ ID NO: 13) |
| PP4 (1085 bp) | forward primer 5'-GCTCAGCCATTCTCAGGAAC-3'; (SEQ ID NO: 14) |
| | reverse primer 5'-GCCATTGTCCCAGTCAAGAT-3'(SEQ ID NO: 15) |
| PP5 (1011 bp) | forward primer 5'-TCGCGTCAAGAGGGTATTTT-3'; (SEQ ID NO: 16) |
| | reverse primer 5'-TAGGACCCATGGCTCTACCC-3'(SEQ ID NO: 17) |
| PP6 (1098 bp) | forward primer 5'-CACGAGTCAGGTGGGAAACT-3'; (SEQ ID NO: 18) |
| | reverse primer 5'-GACGTCTGCTGCTTTTCTGC-3'(SEQ ID NO: 19) |
| PP7 (963 bp) | forward primer 5'-AGGCACGGACTAGCAGGAC-3'; (SEQ ID NO: 20) |
| | reverse primer 5'-ATGCGGACGAAGCCAGAG-3'(SEQ ID NO: 21) |
| PP8 (986 bp) | forward primer 5'-TTAGCATTCAGCCCCTCTGT-3'; (SEQ ID NO: 22) |
| | reverse primer 5'-TTCATGAGATGCAGTCAGCAG-3'(SEQ ID NO: 23) |
| PP9 (951 bp) | forward primer 5'-ACAACTGGTAGGGGCAACAG-3'; (SEQ ID NO: 24 |
| | reverse primer 5'-TGTGTGGCTTTGGCAAATAA-3'(SEQ ID NO: 25) |
| PP10 (990 bp) | forward primer 5'-GCACTGTGCTCCAACTGTGT-3'; (SEQ ID NO: 26) |
| | reverse primer 5'-GGGGGAGTGTTCTTTTCCTT-3'(SEQ ID NO: 27) |

### EXAMPLE 1

### Comparison of Pitx1 expression in osteoblasts of AIS subjects with that in osteoblast of matched controls

To determine whether pitx1 plays a role in the genetic control of AIS development and/or progression, an expression analysis of pitx1 gene using RNA prepared from osteoblasts cultures derived from biopsies obtained intraoperatively of severely affected AIS patients (n=46) and from non-scoliotic patients (trauma cases) control subjects (n=29) was performed.

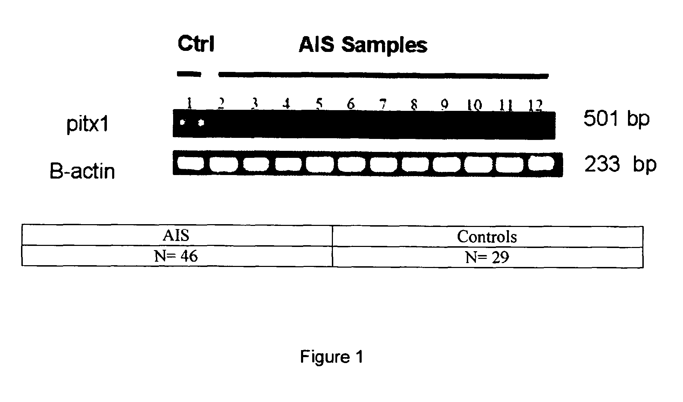

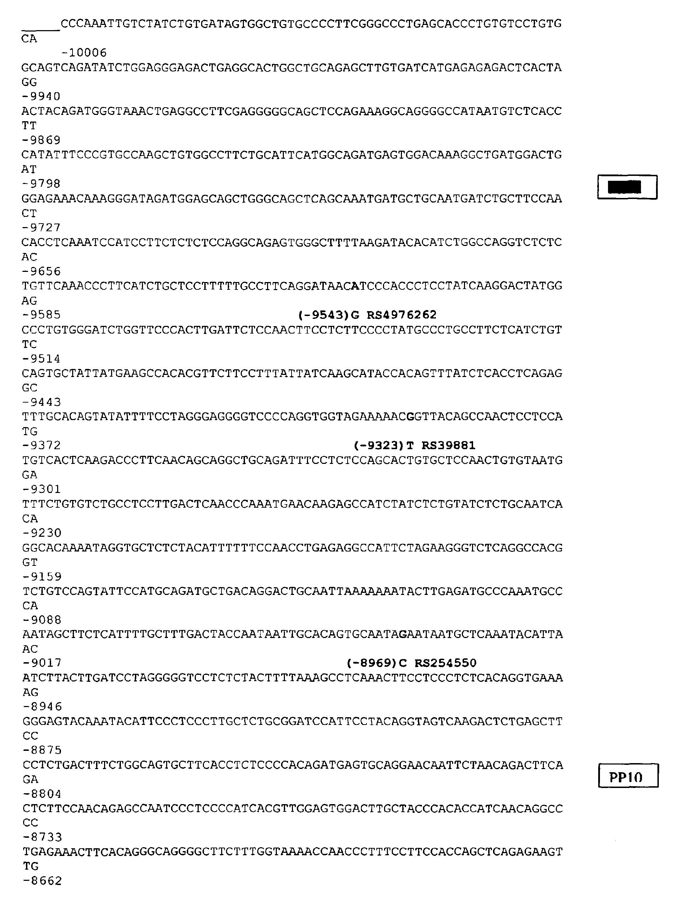

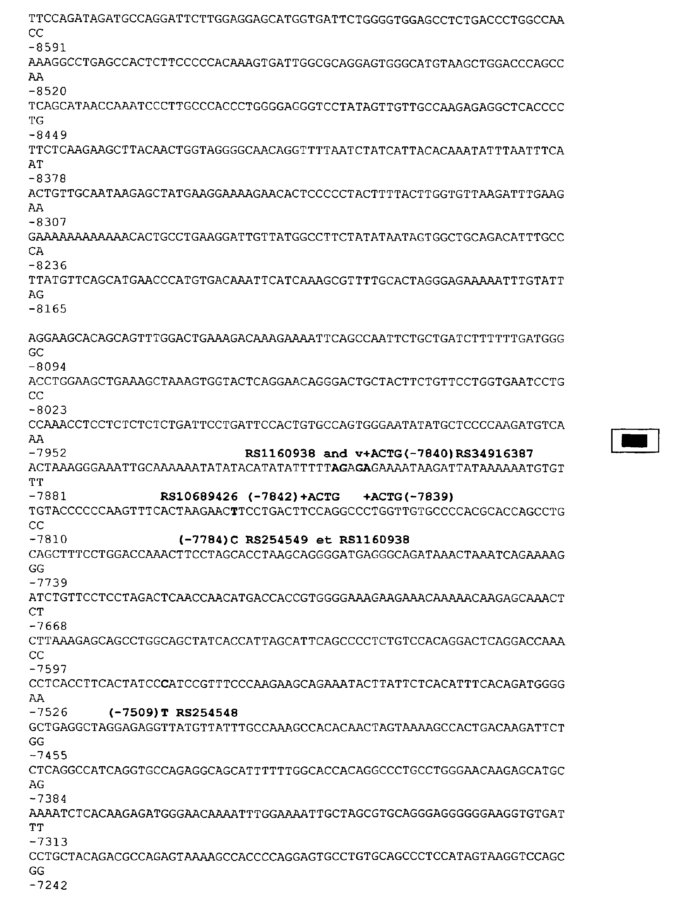

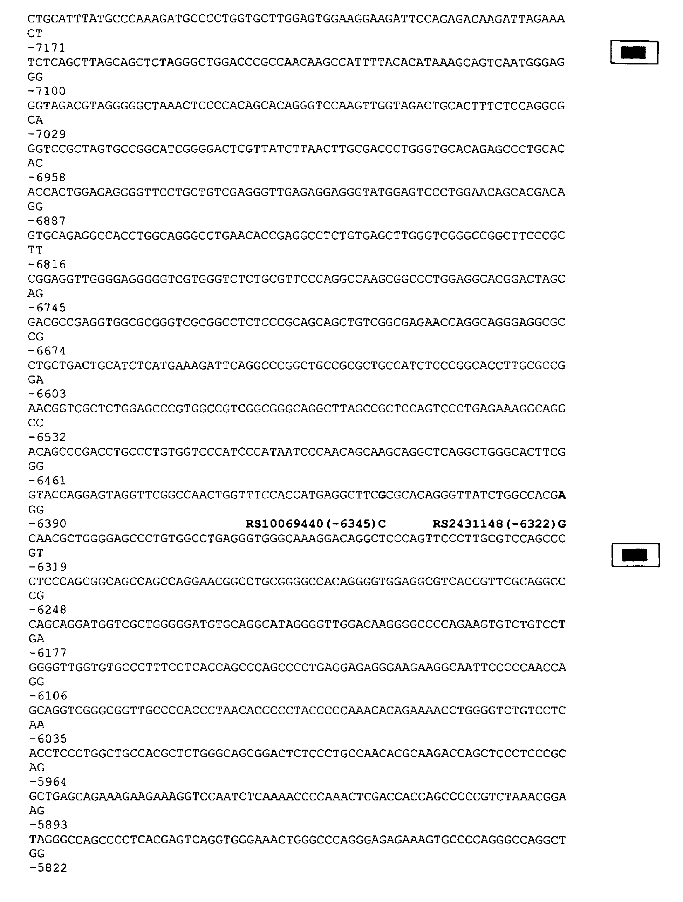

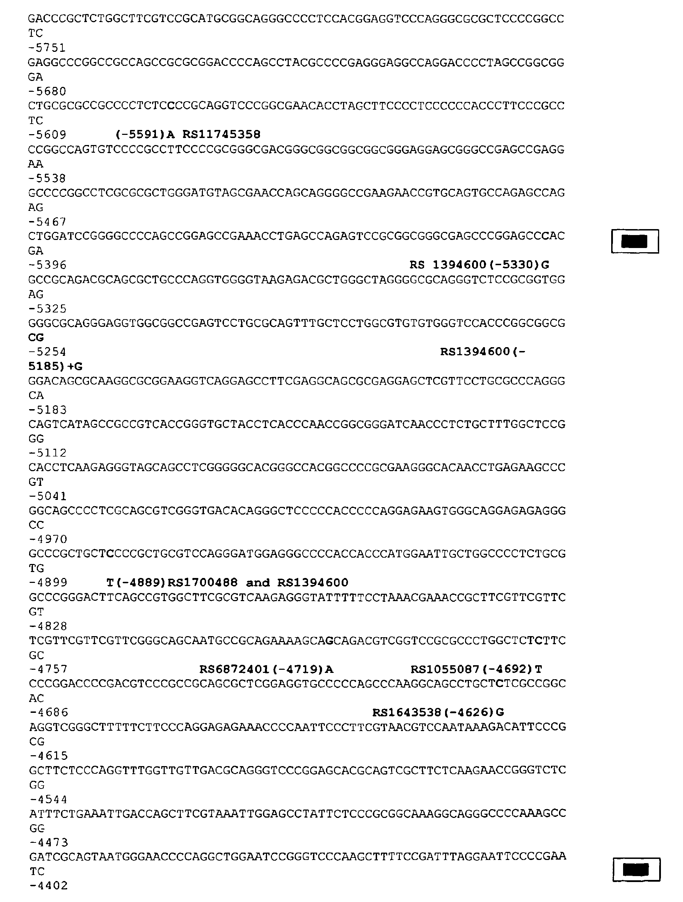

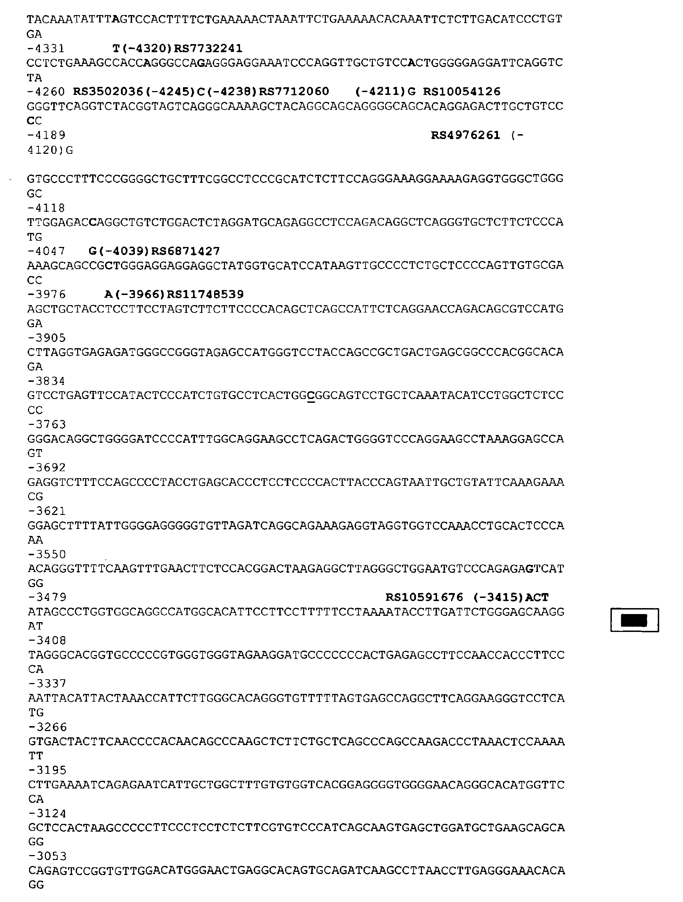

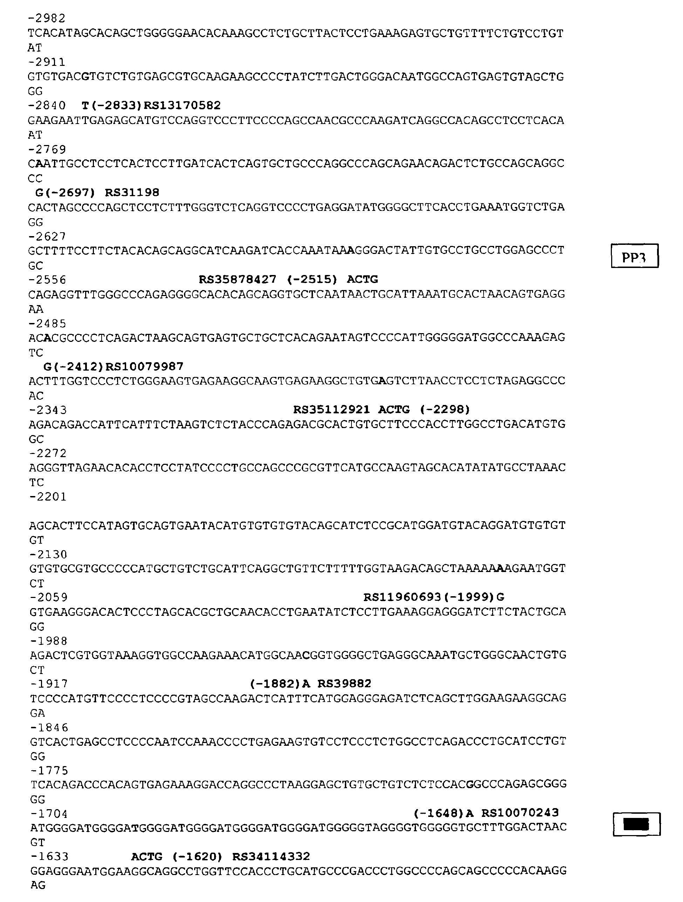

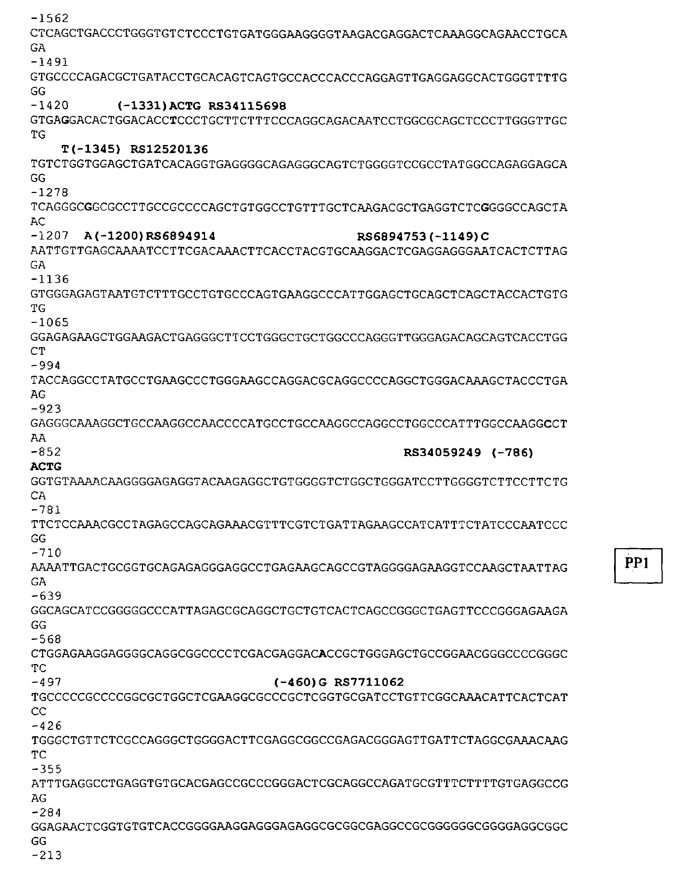

As may be seen in Figure 1, all osteoblasts derived from the AIS patients showed a loss of pitx1 mRNA expression, while control subjects still expressed the mRNA.

### EXAMPLE 2

### Determination of Pitx1 expression in a subject sample

Tissue such as muscle (using for instance a needle in the paraspinal region), bone, cartilage, peripheral blood mononuclear cells (PBMCs such as T and B lymphocytes as well as macrophages) or any cells derived from tissues where Pitx1 is expressed is isolated from the patient. Extraction of RNA from these tissues is done using any standard RNA extraction method such as the standard Trisol Reagent method. Coding region of Pitx1 501 bp in length is amplified from the cDNA using for instance RT-PCR or real-time PCR. The sense primer 5'- GACCCAGCCAAGAAGAAGAA-3' (SEQ ID NO: 6) and the antisense primer 5'- GAGGTTGTTGATGTTGTTGAGG-3' (SEQ ID NO: 7) under the following PCR conditions: Enzyme used: Taq DNA polymerase from Invitrogen™. PCR conditions: 95°C 5minutes Hot start (1 cycle). Following three reactions (32 cycles): 94°C 45 Seconds Denaturation; 55°C 45 Seconds Primer annealing; 72°C 1 minute Elongation; 72°C 2 minutes Last elongation (1 cycle); and 4°C 20 minutes pause. Duration: 2 hours 42 minutes.

## Claims

1. A method of stratifying a subject having adolescent idiopathic scoliosis (AIS) comprising: providing a cell sample isolated from the subject; detecting Paired-like homeodomain transcription factor 1 (Pitx1) expression in the cell sample; whereby the results of the detecting step enables the stratification of the subject having AIS as belonging to an AIS subclass.

2. A method of stratifying a subject having adolescent idiopathic scoliosis (AIS) for a clinical trial comprising: providing a cell sample isolated from the subject; detecting Paired-like homeodomain transcription factor 1 (Pitx1) expression in the cell sample; and stratifying the subject for a clinical trial based on the results of the detecting step.

3. A method for predicting a risk for developing adolescent idiopathic scoliosis (AIS) in a subject comprising
providing a cell sample isolated from the subject; and
detecting Paired-like homeodomain transcription factor 1 (Pitx1) expression in the cell sample;
wherein an absence of Pitx1 expression is indicative that the subject is at risk for developing AIS.

4. The method of claim 3, wherein an absence of Pitx1 expression is indicative that the subject is at risk for developing a Cobb's angle of 45° and above.

5. The method of any one of claims 1 to 4 wherein the detecting step is performed with (a) an isolated nucleic acid molecules specific to a Pitx1 transcription product; or (b) an antibody that binds specifically to Pitx1.

6. The method of claim 5, wherein the isolated nucleic acid molecule is detectably labeled.

7. The method of any one of claims 1 to 6, wherein the cell sample is selected from the group consisting of an osteoblasts sample, a chondrocytes sample, a skeletal myoblasts sample and a blood sample.

8. The method of any one of claims 1 to 6, wherein the cell sample is an osteoblasts sample.

9. The method of any one of claims 1 to 8, further comprising a step of selecting a preventive action or a treatment in light of the results of the detecting step.

10. The method of claim 3 or 4, wherein said subject is pre-diagnosed as being a likely candidate for developing adolescent idiopathic scoliosis.

11. A method of selecting a compound potentially useful in the treatment of adolescent idiopathic scoliosis, said method comprising the steps of
(a) contacting a test compound with at least one cell known to express Paired-like homeodomain transcription factor 1 (Pitx1); and
(b) determining Pitx1 expression level;
wherein the test compound is selected if the Pitx1 expression level is increased in the presence of the test compound as compared to that in the absence thereof.

12. The method of claim 11, wherein said cell is an osteoblast.

13. The method of claim 11 or 12, wherein said cell is from a subject having adolescent idiopathic scoliosis (AIS).

14. The method of any one of claims 1 10 wherein the subject is a human.

## Patentansprüche

1. Verfahren zur Stratifizierung eines Individuums mit adoleszenter idiopathischer Skoliose (AIS), das Folgendes umfasst: Bereitstellen einer Zellprobe, die von dem Individuum isoliert wurde; Nachweisen der Expression des Transkriptionsfaktors Pitx1 (paired-like homeodomain 1) in der Zellprobe; wobei die Ergebnisse des Nachweisschrittes die Stratifizierung des Individuums mit AIS bezüglich der Zugehörigkeit zu einer Subklasse der AIS ermöglichen.

2. Verfahren zur Stratifizierung eines Individuums mit adoleszenter idiopathischer Skoliose (AIS) für eine klinische Studie, das Folgendes umfasst: Bereitstellen einer Zellprobe, die von dem Individuum isoliert wurde; Nachweisen der Expression des Transkriptionsfaktors Pitx1 (paired-like homeodomain 1) in der Zellprobe und Stratifizierung des Individuums für eine klinische Studie auf der Grundlage der Ergebnisse von dem Nachweisschritt.

3. Verfahren zur Vorhersage eines Risikos für die Entwicklung von adoleszenter idiopathischer Skoliose (AIS) bei einem Individuum, das Folgendes umfasst:
Bereitstellen einer Zellprobe, die von dem Individuum isoliert wurde; und
Nachweisen der Expression des Transkriptionsfaktors Pitx1 (paired-like homeodomain 1) in der Zellprobe;
wobei eine Abwesenheit der Pitx1-Expression darauf hinweist, dass ein Risiko für die Entwicklung von AIS für das Individuum vorhanden ist.

4. Verfahren nach Anspruch 3, wobei eine Abwesenheit der Pitx1-Expression darauf hinweist, dass ein Risiko für die Entwicklung eines Cobb-Winkels von 45° und größer für das Individuum vorhanden ist.

5. Verfahren nach einem der Ansprüche 1 bis 4, wobei der Nachweisschritt (a) mit isolierten Nukleinsäuremolekülen, die für ein Pitx1-Transkriptionsprodukt spezifisch sind, oder (b) mit einem Antikörper, der spezifisch an Pitx1 bindet, durchgeführt wird.

6. Verfahren nach Anspruch 5, wobei das isolierte Nukleinsäuremolekül nachweisbar markiert ist.

7. Verfahren nach einem der Ansprüche 1 bis 6, wobei die Zellprobe ausgewählt ist aus der Gruppe, die aus einer Osteoblasten-Probe, einer Chondrozyten-Probe, einer Myoblasten-Probe aus Skelettmuskulatur und einer Blutprobe besteht.

8. Verfahren nach einem der Ansprüche 1 bis 6, wobei die Zellprobe eine Osteoblasten-Probe ist.

9. Verfahren nach einem der Ansprüche 1 bis 8, das ferner einen Schritt des Auswählens einer präventiven Maßnahme oder einer Behandlung in Anbetracht der Ergebnisse des Nachweisschrittes umfasst.

10. Verfahren nach Anspruch 3 oder 4, wobei das Individuum bereits als ein möglicher Kandidat für die Entwicklung einer adoleszenten idiopathischen Skoliose diagnostiziert wurde.

11. Verfahren zum Auswählen einer Verbindung, die möglicherweise für die Behandlung der adoleszenten idiopathischen Skoliose zweckmäßig ist, wobei das Verfahren die Schritte von
(a) dem Inkontaktbringen einer Testverbindung mit mindestens einer Zelle, von der bekannt ist, dass sie den Transkriptionsfaktor Pitx1 (paired-like homeodomain 1) exprimiert; und
(b) dem Bestimmen der Expressionsstärke von Pitx1 umfasst;
wobei die Testverbindung ausgewählt wird, wenn die Pitx1-Expressionsstärke in Anwesenheit der Testverbindung im Vergleich zu derjenigen in Abwesenheit der Testverbindung erhöht ist.

12. Verfahren nach Anspruch 11, wobei die Zelle ein Osteoblast ist.

13. Verfahren nach Anspruch 11 oder 12, wobei die Zelle von einem Individuum mit adoleszenter idiopathischer Skoliose (AIS) ist.

14. Verfahren nach einem der Ansprüche 1 bis 10, wobei das Individuum ein Mensch ist.

## Revendications

1. Méthode de stratification d'un sujet ayant une scoliose idiopathique de l'adolescent (SIA) comprenant : l'obtention d'un échantillon cellulaire isolé chez le sujet ; la détection de l'expression du Facteur de transcription à homéodomaine de type Paired 1 (Pitx1) dans l'échantillon cellulaire ; dans laquelle les résultats de l'étape de détection permettent la stratification du sujet ayant une SIA comme faisant partie d'une sous-classe des SIA.

2. Méthode de stratification d'un sujet ayant une scoliose idiopathique de l'adolescent (SIA) pour un essai clinique comprenant : l'obtention d'un échantillon cellulaire isolé chez le sujet ; la détection de l'expression du Facteur de transcription à homéodomaine de type Paired 1 (Pitx1) dans l'échantillon cellulaire ; et la stratification du sujet pour un essai clinique basé sur les résultats de l'étape de détection.

3. Méthode de prédiction des risques pour un sujet de développer une scoliose idiopathique de l'adolescent (SIA) comprenant
l'obtention d'un échantillon cellulaire isolé chez le sujet ; et
la détection de l'expression du Facteur de transcription à homéodomaine de type Paired 1 (Pitx1) dans l'échantillon cellulaire ;
dans laquelle l'absence d'expression de Pitx1 est une indication que le sujet est à risque pour le développement d'une SIA.

4. Méthode selon la revendication 3, dans laquelle une absence d'expression de Pitx1 est une indication que le sujet est à risque pour le développement d'un angle de Cobb de 45° et plus.

5. Méthode selon l'une des revendications 1 à 4, dans laquelle l'étape de détection est réalisée avec (a) une molécule d'acide nucléique isolée spécifique d'un produit de transcription de Pitx1 ; ou (b) un anticorps qui se lie spécifiquement à Pitx1.

6. Méthode selon la revendication 5, dans laquelle la molécule d'acide nucléique isolée est marquée de manière détectable.

7. Méthode selon l'une des revendications 1 à 6, dans laquelle l'échantillon cellulaire est choisi dans le groupe constitué d'un échantillon d'ostéoblastes, d'un échantillon de chondrocytes, d'un échantillon de myoblastes squelettique et d'un échantillon sanguin.

8. Méthode selon l'une des revendications 1 à 6, dans laquelle l'échantillon cellulaire est un échantillon d'ostéoblastes.

9. Méthode selon l'une des revendications 1 à 8, comprenant en outre une étape de sélection d'une action préventive ou un traitement à la lumière des résultats de l'étape de détection.

10. Méthode selon la revendication 3 ou 4, dans laquelle ledit sujet est prédiagnostiqué comme étant un candidat probable pour développer une scoliose idiopathique de l'adolescent.

11. Méthode de sélection d'un composant potentiellement utile dans le traitement d'une scoliose idiopathique de l'adolescent, ladite méthode comprenant les étapes de
(a) la mise en contact d'un composé test avec au moins une cellule connue pour exprimer le Facteur de transcription à homéodomaine de type Paired 1 (Pitx1) ; et
(b) la détermination du niveau d'expression de Pitx1 ;
dans laquelle le composé test est choisi si le niveau d'expression de Pitx1 est augmenté en présence du composé test par rapport au niveau en son absence.

12. Méthode selon la revendication 11, dans laquelle ladite cellule est un ostéoblaste.

13. Méthode selon la revendication 11 ou 12, dans laquelle ladite cellule provient d'un sujet ayant une scoliose idiopathique de l'adolescent (SIA).

14. Méthode selon l'une des revendications 1 à 10, dans laquelle le sujet est un humain.
